# EUROPEAN PATENT SPECIFICATION

(11) **EP 2 353 490 B1**
(45) Date of publication and mention of the grant of the patent: **10.07.2019**
(21) Application number: 10195976.5
(22) Date of filing: 20.12.2010
(51) Int. Cl.: A61B 1/00, G02B 6/38, H01R 13/631

(54) **Medical equipment and endoscope apparatus**
Medizinische Vorrichtung und Endoskopvorrichtung
Équipement médical et appareil d'endoscope

(30) Priority: 28.01.2010 JP 2010017487
(43) Date of publication of application: 10.08.2011
(73) Proprietor: Fujifilm Corporation, Minato-ku Tokyo (JP)
(72) Inventor: KAGAYA, Hiroto, Kanagawa (JP); MIZOYOSHI, Akira, Kanagawa (JP); IIDA, Takayuki, Kanagawa (JP)
(74) Representative: Klunker IP Patentanwälte PartG mbB

(56) References cited:
- EP-A2- 1 989 989
- US-A- 5 157 749
- US-A1- 2007 088 198
- US-A1- 2008 281 157

## Description

### 1. Technical Field

The invention relates to a medical equipment according to the preamble of claim 1, and an endoscope apparatus.

### 2. Description of Related Art

A medical equipment of this type is known from e.g. EP1990871.

There is an endoscope apparatus including an endoscope having an illumination optical system that emits illumination light from a leading end of an endoscope insertion portion to be inserted into an object to be examined, and a control apparatus to which the endoscope is connected. The control apparatus has a light source device that generates the illumination light and a video processor that performs image processing. A universal cord is connected to a main body portion of the endoscope. The universal cord is provided therein with signal lines that are connected to the illumination optical system and an observation optical system, a bending operation wire, and an air-supply/water-supply channel and/or a suction channel. The universal cord is connected to each control apparatus such as the light source device through a connector portion. The connector portion, which is connected to the light source device, is provided with optical connectors. The optical connectors are configured to constitute a pair capable of connecting a plug of the endoscope and a receptacle of the light source device (for example, see JP 2008-278971 A (corresponding to US 2008/0281157 A).

Recently, an endoscope diagnosis has been performed which captures a fine lesion with a special light observation. The special light observation includes a narrow band light observation that enhances and displays superficial blood vessels, a fluorescence observation that observes autofluorescence of a living body, an infrared light observation that extracts information of deep blood vessels using fluorescence from administered medical agent, and the like. While white light illumination is used for a normal observation, light having a wavelength of 405 nm, for example, is used for the narrow band light observation and fluorescence observation, and light having a wavelength of 760 nm, for example, is used for the infrared light observation. Also, light having a wavelength of 405 nm, for example, is used for photodynamic diagnosis (PDD), and light having a wavelength of 630 nm, for example, is used for photodynamic therapy (PDT). Accordingly, when performing ones of the above observations and treatment, a plurality of optical fibers passes through the universal cord.

Therefore, the plug and the receptacle, which connect the optical fibers, have a plurality of pairs of a plug-side holder and a receptacle-side holder for connecting the optical fibers. The plug-side holder maintains the plug-side optical fiber and the receptacle-side holder maintains the receptacle-side optical fiber. However, in order to collectively connect the plurality of pairs of plug-side holder and the receptacle-side holder, it is necessary to highly precisely assemble the plug-side and the receptacle-side, which causes difficulty in manufacturing and/or handling. Also, a simple plug connection operation of just inserting the plug into the receptacle may cause damage or deviation of an optical axis due to shock in connecting the plug and the receptacle, depending on the insertion operation of the plug. Thus, it is, in general, difficult to stably match the optical axes of all optical fibers with high precision all the time.

### SUMMARY OF THE INVENTION

The invention, which is defined in claim 1, has been made in view of the above circumstances, and may provide a medical equipment capable of precisely connecting optical fibers with a simple operation and realizing optical connection with low loss and may stably guide laser light all the time.

With the above medical equipment and endoscope apparatus, when the optical connector is connected, the plurality of engagement pairs having different engagement beginning positions are sequentially engaged, so that it is possible to increase the positioning precision step by step. When the connection of the optical connector is completed, optical axes of the optical fibers are automatically matched with high precision. Therefore, it is possible to simply connect the optical fibers with low loss, so that it is possible to stably guide laser light.

### BRIEF DESCRIPTION OF THE DRAWINGS

Fig. 1 is an appearance view showing an example of an endoscope apparatus according to an embodiment of the invention.
Fig. 2 is a conceptual block diagram of the endoscope apparatus.
Fig. 3 is a perspective view of a plug.
Fig. 4 is a section view taken along an axis line of the plug.
Fig. 5 is a perspective view of a receptacle.
Fig. 6 is a section view taken along an axis line of the receptacle.
Fig. 7A is a perspective view of a floating base having receptacle-side holders attached thereto when viewed from a diagonally front side.
Fig. 7B is a perspective view of the floating base when viewed from a diagonally rear side.
Fig. 8 is a perspective view of holder alignment units shown in Fig. 7.
Fig. 9 is a perspective view of a plug-side holder and the receptacle-side holder just before connection.
Fig. 10 is a perspective view of the plug and the receptacle in which a cam cylinder is shown in an exposed manner and is engaged with engagement pins.
Fig. 11A is a section view showing a connection configuration of optical fibers before connection.
Fig. 11B is a section view showing a connection configuration of the optical fibers after connection.
Fig. 12 is a section view of the plug and the receptacle before connection begins.
Fig. 13 is a graph showing a relation between an axial distance and a radial tolerance at each engagement portion together with a schematic view of main parts.
Fig. 14 is a section view of the plug and the receptacle with the engagement pins being in a contact state.
Fig. 15 is a section view of the plug and the receptacle before the engagement pins are inserted.
Fig. 16 illustrates a core alignment operation by displacement of second linear support pieces.
Fig. 17 illustrates a core alignment operation by displacement of first linear support pieces.
Fig. 18 is a perspective view showing displacement directions of the receptacle-side holder that is supported and is subject to the core alignment operation by a holder alignment unit.
Fig. 19 is a section view of the plug and receptacle after the engagement pins are inserted.
Fig. 20 illustrates a light transmission state that is changed depending on lengths of a plug-side stub and a receptacle-side stub.
Fig. 21 is a graph showing a tolerance in an optical axis direction.
Fig. 22 is a graph showing a tolerance in a direction perpendicular to the optical axis.
Fig. 23 is a schematic section view showing another connection configuration of a plug and a receptacle.
Fig. 24 is a perspective view of holder alignment units according to a modified embodiment in which a receptacle-side holder is fixed to first linear support pieces via a vertical support plate.
Fig. 25 is a perspective view of holder alignment units according another modified embodiment in which first linear support pieces and second linear support pieces extend in an opposite direction.

### DETAILED DESCRIPTION OF EMBODIMENTS

Hereinafter, embodiments of the invention will be described with reference to the accompanying drawings.

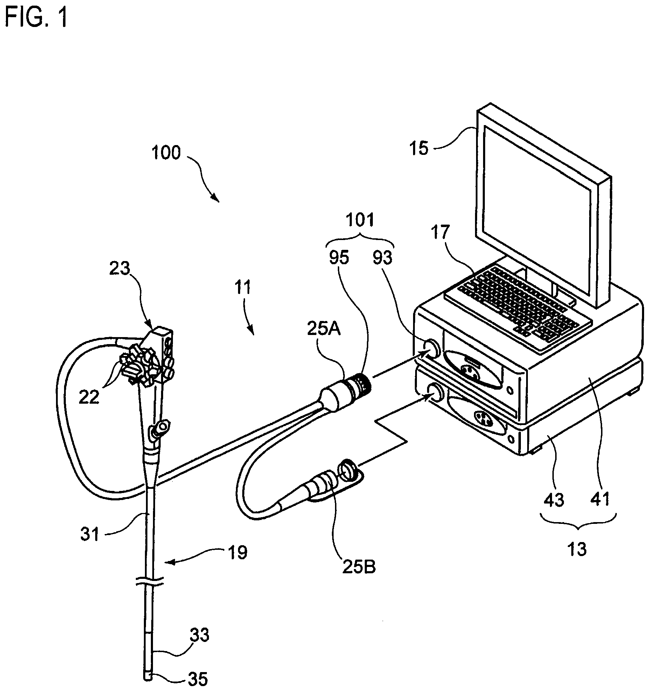

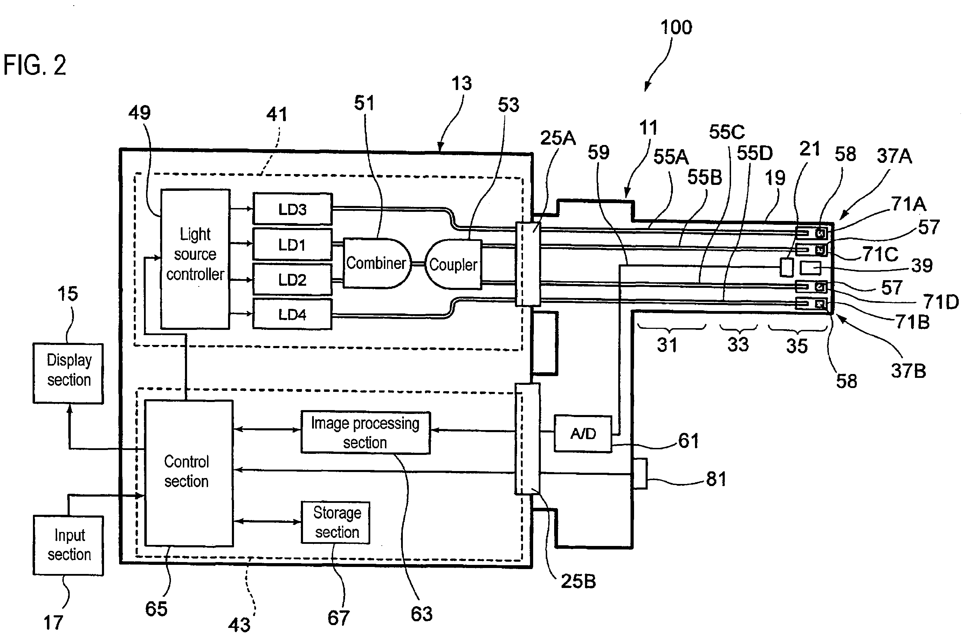

Fig. 1 is an appearance view showing an example of an endoscope apparatus according to an embodiment of the invention. Fig. 2 is a conceptual block diagram of the endoscope apparatus.

As shown in Figs. 1 and 2, an endoscope apparatus 100 that is one medical equipment has an endoscope 11 and a control device 13 to which the endoscope 11 is connected. The control device 13 is connected with a display section 15 that displays image information and an input section 17 that receives an input operation. The endoscope 11, which is an electronic endoscope, has an illumination optical system that emits illumination light from a leading end of an endoscope insertion portion 19, which is inserted into an object to be examined, and an imaging optical system that includes an imaging device which images an area to be observed.

Also, the endoscope 11 has the endoscope insertion portion 19, an operation section 23, connector sections 25A, 25B. The operation section 23 is used to perform an operation of bending the leading end of the endoscope insertion portion 19 and an observation operation. The connector sections 25A, 25B detachably connect the endoscope 11 to the control device 13. Although not shown, a variety of channels such as forceps channel for inserting a treatment tool for collecting tissue and air supply/water supply channels are provided in the operation section 23 and the endoscope insertion portion 19.

The endoscope insertion portion 19 has a flexible part 31 having flexibility, a bending part 33, and a leading end part 35 (hereinafter, which may be referred to as an "endoscope leading end part"). The endoscope leading end part 35 is provided with irradiation ports (which will be described in detail later) through which light is irradiated to an area to be observed and an imaging device 21 (refer to Fig. 2), which obtains image information of the area to be observed, such as a CCD (Charge Coupled Device) image sensor and a CMOS (Complementary Metal-Oxide Semiconductor) image sensor. Also, an object lens unit is arranged on a light receiving surface side of the imaging device 21.

The bending part 33 is provided between the flexible part 31 and the leading end part 35. Te bending part 33 can be bent by a rotation operation performed by an angle knob 22, which is disposed in the operation section 23. The bending part 33 can be bent in an arbitrary direction at an arbitrary angle, depending on a part of an object to be examined for which the endoscope 11 is used, thereby enabling the irradiation ports of the endoscope leading end part 35 and an observation direction of the imaging device 21 to be directed toward a desired observation part.

The control device 13 has a light source device 41 and a processor 43. The light source device 41 generates light to be supplied to the irradiation ports of the endoscope leading end part 35. The processor 43 performs image processing for an image signal from the imaging device 21. The control device 13 is connected to the endoscope 11 via the connector sections 25A, 25B. Also, the processor 43 is connected to the display section 15 and the input section 17. The processor 43 performs the image processing for the imaging signal transmitted from the endoscope 11 in accordance with a command from the operation section 23 of the endoscope 11 or input section 17 and generates and supplies an image for display to the display section 15,.

As shown in Fig. 2, the light source device 41 has a plurality of laser light sources having different light emitting wavelengths. In this illustrative embodiment, the light source device 41 has a laser light source LD1 having a central wavelength of 405 nm, a laser light source LD2 having a central wavelength of 445 nm and laser light sources LD3, LD4 having a central wavelength of 405 nm. The laser light source LD1 is a light source for narrow band light observation that emits violet laser light. The laser light source LD2 is a light source for normal observation that emits blue laser light and thus generates white illumination light by a wavelength conversion member (which will be described later). Also, the laser light sources LD3, LD4 are light sources for fluorescence observation and are configured to emit light toward an area to be observed without the light passing through a phosphor (which will be described later).

Also, in this configuration, the laser light sources LD3, LD4 may share a common light path, and a laser light source LD (not shown) having a central wavelength of 472 nm, a laser light source LD (not shown) having a central wavelength of 665 nm and a laser light source LD (not shown) having a central wavelength of 785 nm may be further provided. The Laser light having the central wavelength of 472 nm, which is emitted from the laser light source LD (not shown) sharing the common light path with the laser light sources LD3, LD4 is used for extracting information about blood oxygen saturation and a blood vessel depth. The laser light having the central wavelength of 665 nm is laser light for therapy and is used in the photodynamic therapy (PDT) of irradiating the laser light to a living tissue surface with a relatively strong output and thus treating a tumor such as cancer. Further, the laser light having the central wavelength of 785 nm is used in infrared observation of ICG (indocyanine green) administered into the blood vessels.

Also, the laser light source LD1 can be used as an illumination light source for the photodynamic diagnosis (PDD). The PDD is a diagnosis method of administering a photosensitive material, which has a tumor affinity property and responds to specific excitation light, into the body in advance, and irradiating laser light, which is excitation light, to a living tissue surface with a relatively weak output and observing fluorescent light from a part in which a concentration of the photosensitive material is increased, such as a lesion part in which the tumor such as cancer exists. The PDT is performed for the lesion part specified by the PDD.

The light from the respective laser light sources LD1 to LD4 (and laser light sources (not shown) sharing the common light path with the light paths of the laser light sources LD3, LD4) are individually controlled by a light source controller 49. The respective laser light can be generated individually or simultaneously. Also, emitting timings of the respective laser light sources and a ratio of amounts of light emitted from the respective laser light sources can be arbitrarily changed by operating a switch 81 of the endoscope 11, by an operation of the input section 17, or by the light source device 41.

As the laser light sources LD1 to LD4, InGaN-based laser diodes of a broad area type, InGaNAs-based laser diodes and GaNAs-based laser diodes can be used. Also, a semiconductor light emitting device such as light emitting diode can be used as the light sources. In place of the semiconductor light emitting device, light obtained by wavelength-selecting, by a color filter, light emitted from a white light source such as xenon lamp can be used.

The laser light emitted from the laser light sources LD1 to LD4 are respectively introduced into optical fibers by condenser lenses. The laser light from the laser light sources LD1, LD2 are combined by a combiner 51 shown in Fig. 2, divided by a coupler 53 and then transmitted to the connector section 25A. Thereby, the laser light from the laser light sources LD1, LD2 are uniformly transmitted to optical fibers 55B, 55C while non-uniformity of the light emitting wavelengths or speckle due to a difference of the individual laser light sources is reduced. In the meantime, it may be possible that the laser light from the laser light sources LD1, LD2 are directly transmitted to the connector section 25A without using the combiner 51 and coupler 53, which can simplify the configuration of the light source device.

The optical fibers 55A to 55D are multi-mode fibers. As the fibers, a thin fiber cable having a core diameter of 105 µm, a clad diameter of 125 µm and a diameter ϕ of 0.3 to 0.5 mm, which includes a protective layer that is an outer cover, may be used, for example. In the meantime, the endoscope apparatus 100 may use a single mode fiber that transmits the light in a single mode only.

The laser light from the laser light sources LD1 to LD4 are introduced at any timings into the optical fibers 55A to 55D that extend from the connector section 25A to the endoscope leading end part 35. The laser lights from the laser light sources LD1, LD2 are transmitted to a phosphor 57 that is arranged at the endoscope leading end part 35. The laser lights from the laser light sources LD3, LD4 are transmitted to light deflection/diffusion members 58. Then, the laser lights are emitted as illumination lights (or therapy lights) to an area to be observed through the irradiation ports 37A, 37B.

Here, the optical fiber 55A and the light deflection/diffusion member 58 constitute a light irradiation unit 71A. The optical fiber 55B and the light deflection/diffusion member 58 constitute a light irradiation unit 71B. The optical fiber 55B and the phosphor 57 constitute a light irradiation unit 71C. The optical fiber 55C and the phosphor 57 constitute a light irradiation unit 71D. The light irradiation units 71A, 71C are disposed across the imaging device 21 and object lens unit 39 of the endoscope leading end part 35, and the light irradiation units 71B, 71D are also disposed across the imaging device 21 and object lens unit 39 of the endoscope leading end part 35.

The phosphors 57 of the light irradiation units 71C, 71D include a plurality of fluorescent substances (for example, YAG-based fluorescent substances or BAM (BaMgAl₁₀O₁₇)), which absorb a part of the blue laser light from the laser light source LD2 and thus excitedly emit lights of green to yellow. Thereby, the excitedly emitted lights of green to yellow, which is excited by the excitation blue laser light, and the blue laser light, which transoms through the phosphors without being absorbed by the phosphors 57, are combined to generate white (pseudo-white) illumination light.

The blue laser light is indicated by an emission line having a central wavelength of 445 nm. The excitedly emitted light, which results from the blue laser light, from the phosphor 57 forms a spectrum intensity distribution which has large luminescence intensity in a wavelength band of about 450 nm to 700 nm. The white light is generated by profiles of the excitedly emitted light and the blue laser light. Where the semiconductor light emitting devices are used as the excitation light sources as in this illustrative embodiment, it is possible to obtain the white light of high intensity in high luminescence efficiency. Therefore, it is possible to easily adjust the intensity of the white light and to suppress a change in color temperature and chromaticity of the white light.

Here, the white light described in the specification is not strictly limited to light including all wavelength components of the visible lights, but is any light so long as it includes light of specific wavelength bands of R (red), G (green) and B (blue) that are primary colors. For example, in a broad sense, the white light may be light having wavelength components from green to red or light having wavelength components from blue to green.

The phosphors 57 can prevent noise superposition, which is an obstacle to the imaging, or flicker that is generated when displaying a moving picture, which are caused due to speckles generated by coherence of the laser lights. Also, regarding the phosphor 57, it is preferable to configure the fluorescent substance and a diameter of the filler material with materials in which light of an infrared region is little absorbed and highly scattered, taking into consideration a difference of refractive indices of a fluorescent substance configuring the phosphor and a resin for fixing and solidification becoming a filler material. Thereby, it is possible to increase a scattering effect without decreasing the intensity of light of red or infrared region, so that an optical loss is reduced.

Further, the light deflection/diffusion members 58 of the light irradiation units 71A, 71B may be made of any material so long as the laser lights from the laser light sources LD3, LD4 can pass through it. For example, a light transmitting resin material or glass may be used. Also, the light deflection/diffusion members 58 may be provided with a light diffusion layer in which particles (filler and the like) having minute unevenness or different refractive indexes are mixed in a surface of a resin material or glass, or may be configured by a semitransparent material. Thereby, the light transmitted from the light deflection/diffusion member 58 becomes light of a narrow band wavelength having an amount of light that is uniform in a predetermined irradiation area.

As described above, the white light, which is generated from the blue laser light and the light excitedly emitted from the phosphors 57, and the narrow band lights emitted from the respective laser lights are irradiated toward an area to be observed of an object to be examined from the leading end part 35 of the endoscope 11. Regarding the area to be observed to which the illumination light is irradiated, an image of the object to be examined is formed by the object lens unit 39 and thus imaged by the imaging device 21.

After the imaging, an image signal of the captured image output from the imaging device 21 is transmitted to an AID converter 61 through a scope cable 59, which is then converted into a digital signal. The converted signal is input to an image processing section 63 of the processor 43 through the connector section 25B. The image processing section 63 performs various processing for the captured image signal, which is output from the imaging device 21 and converted into the digital signal, such as white balance correction, gamma correction, contour enhancement and color correction. The control section 65 generates an endoscope observation image based on the captured image signal, which is processed in the image processing section 63, and various information. Then, the endoscope observation image is displayed on the display section 15. Further, the captured image signal is stored in a storage section 67 such as a memory or a storage device, as required.

The endoscope apparatus 100 combines the plurality of light irradiation units to irradiate the white light and the narrow band lights. Thereby, it is possible to perform both of (i) the normal observation by the white light and (ii) the special light observation such as narrow band light observation, fluorescence observation and infrared light observation, under good illumination conditions. Also, since it is configured that the narrow band lights for special light observation don't pass through the phosphors 57 which is used to generate white light, it is possible to irradiate the narrow band lights, as they are, in high intensity without accompanying unnecessary fluorescent components.

The light source device 41 can supply the laser lights of the laser light sources LD1 to LD4 simultaneously or alternately. Also, the light source device 41 can switch and supply the laser lights, in synchronous with an imaging frame(s) of the imaging device. When the light source device 41 switches the laser light sources LD1 to LD4 in synchronous with the imaging frame(s), in the case of the special light observation, images under irradiation of the laser lights from one or both of the laser light sources LD1, LD2 are captured in even frames and images under irradiation of the laser lights from the laser light sources LD3, LD4 are captured in odd frames, for example. By superimposing and displaying the odd and even frames as image information of one piece, it is possible to display an image of the fluorescence observation on the observation image of the normal observation at the same time. Thereby, it is possible to perform the endoscope diagnosis more smoothly with higher visibility.

It is also possible to display the images of the even frames and the images of the odd frames at different positions in a display area of the display section 15, respectively, without superimposing the images of the even frames and the images of the odd frames as the image information of one piece. In this case, it is possible to perform an observation or treatment while comparing a lesion part and a treatment part, such as respective checks of the lesion part and the treatment part. In this matter, by supplying the plurality of laser lights to the endoscope 11 at any timing, it is possible to generate an optimized illumination pattern corresponding to what is to be observed by the endoscope 11 and to thus improve the diagnosis precision of the endoscope 11.

### <Configuration of Plug and Receptacle>

Next, the configuration of the connector section 25A will be described in more detail.

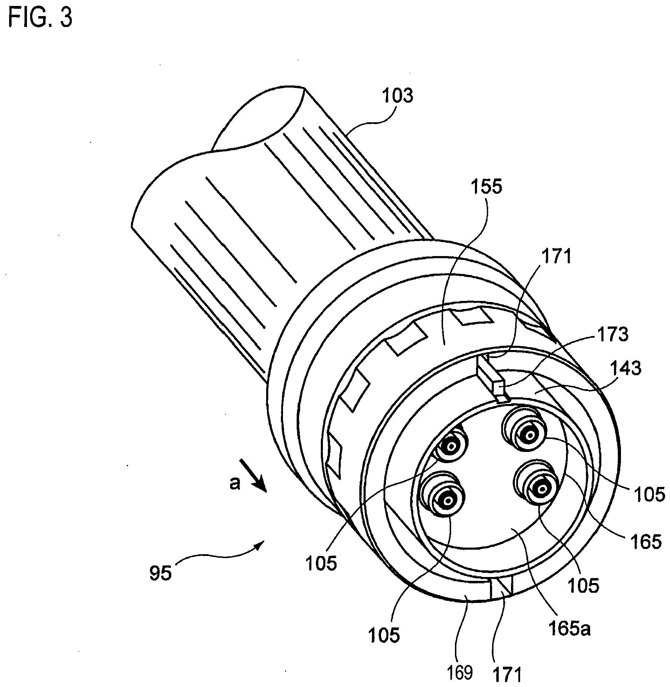

Fig. 3 is a perspective view of a plug 95.

A plug 95 is connected to the optical fibers 55A to 55D (hereinafter, which may be collectively referred to as "plug-side optical fibers" 55), which are connected to the light irradiation units 71A to 71D shown in Fig. 2, and is attached to an end portion of a universal cord 103 including the plug-side optical fibers 55 therein. The plug 95 has a rotatable ring handle 155 on its outer circumference. A metal outer cylinder 143 is provided inside the ring handle 155. The metal outer cylinder 143 is fixed to a plug main body 165.

Four plug-side holders 105 are fixed to a circular connection-side wall part 165a of the plug main body 165. Each of the plug-side holders 105 holds one plug-side optical fiber 55 therein. Also, a cam cylinder 169 that is rotatable with being integrated with the ring handle 155 is fixed on an inner circumference of the ring handle 155. A pair of guide grooves 171 is formed in a leading end surface of the cam cylinder 169. The cam cylinder 169 is integrated with the ring handle 155 and is rotatable with respect to the metal outer cylinder 143. A guide key 173 is provided on the outer circumference of the metal outer cylinder 143 and projects in a plug insertion direction a. The guide key 173 is introduced into a key recess that is provided in a receptacle which is a connection counterpart of the plug 95. It should be noted that the number of the plug-side holders 105 is arbitrary. Namely, the four plug-side holders are exemplified in this illustrative embodiment. Also, although not shown, the connection-side wall part 165a of the plug main body 165 is provided with the channels for air supply/water supply to the endoscope 11, a position regulation pin and the like.

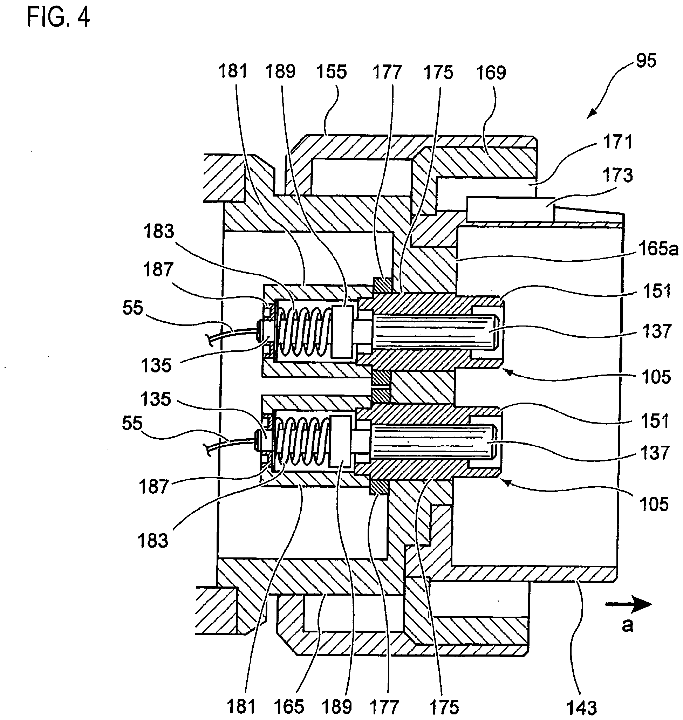

Fig. 4 is a section view taken along an axis line of the plug 95.

The plug-side holder 105 includes a nipple holder 175, a pressing ring 177, a plug-side inner sleeve 137, a cover 181, and a coil spring 183. The plug-side inner sleeve 137 houses a plug-side ferrule 135 therein. The cover 181 covers a base end side of the nipple holder 175. The coil spring 183 is arranged in the cover 181 with the plug-side ferrule 135 serving as an axis core. The nipple holder 175 penetrates through the connection-side wall part 165a of the plug main body 165 and is thus fixed at its outer circumference to the connection-side wall part 165a. The pressing ring 177 is fitted to the outer circumference of the nipple holder 175 and is in contact with a rear surface of the connection-side wall part 165a to restrain the nipple holder 175 from being pulled out in the plug insertion direction a. It is noted that the plug 95 will be described with the plug insertion direction a referred to as front and with a direction opposite to the plug insertion direction a referred to as rear. The cover 181 has a cylindrical shape having a bottom. A leading end opening side of the cover 181 is fixed to a rear end of the nipple holder 175, thereby covering a rear part of the plug-side ferrule 135. A ferrule guide cap 187 is fixed to a rear part of the cover 181. The ferrule guide cap has a through-hole through which a rear end portion of the plug-side ferrule 135 is guided to an outside of the cover 181. The rear end portion of the plug-side ferrule 135, which extends through the ferrule guide cap 187, is connected with the plug-side optical fiber 55.

In the cover 181, a collar part 189 is formed at an approximately central part, in the axial direction, of the plug-side ferrule 135. The coil spring 183 is inserted around the rear part of the plug-side ferrule 135 between the collar part 189 and the ferrule guide cap 187. In other words, the plug-side ferrule 135 is biased in the plug insertion direction a by the coil spring 183. The plug-side ferrule 135 can retreat against the biasing force of the coil spring 183 by having the plug-side ferrule 135 be pressed in the opposite direction to the plug insertion direction a.

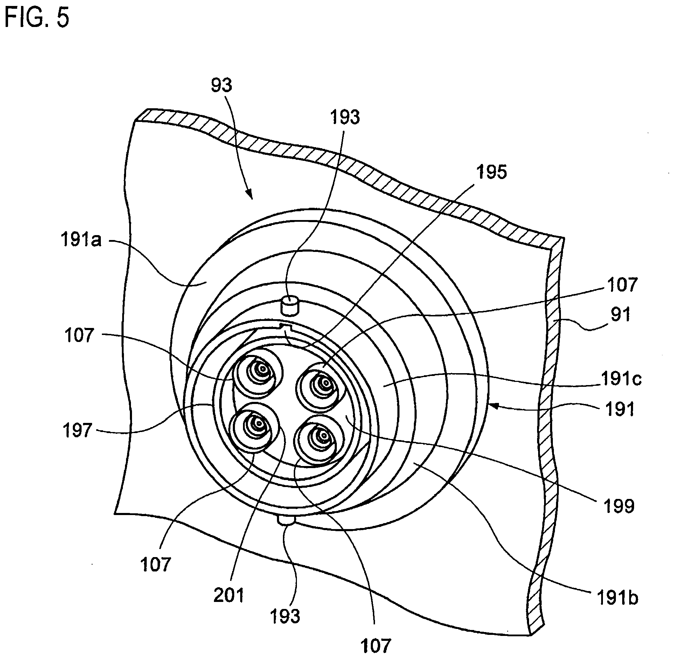

Fig. 5 is a perspective view of a receptacle 93.

The receptacle 93 which is connected to the plug 95 is attached to an equipment main body 91 of the light source device 41 (refer to Fig. 1). In other words, the plug 95 and the receptacle 93 constitute an optical connector 101 that detachably connects the endoscope 11 and the light source device 41. With the above configuration, the illumination light of the endoscope 11 can be simply extracted by connecting the plug 95 to the receptacle 93 of the light source device 41 and can be emitted from the leading end part 35 of the endoscope insertion portion 19. The light source device 41 securely supplies laser lights having different spectra to the endoscope 11 through the optical connector 101, with low optical connection loss.

The receptacle 93 has a metal housing 191 including a flange part 191a, a wedge-shaped part 191b and a leading end cylinder part 191c. A pair of engagement pins 193, which protrude in a diametrical direction, is fixed in an outer circumference of the leading end cylinder part 191c. The engagement pins 193 are adapted to be engaged with the guide grooves 171, which are formed in the cam cylinder 169 of the plug 95 (refer to Fig. 3). A key groove 195 is formed at a rear-side position of the engagement pin 193 on an inner circumference of the leading end cylinder part 191c. The key groove 195 is adapted to house the guide key 173, which is provided in the metal outer cylinder 143 of the plug 95.

At an axial position which is 25 mm before the plug 95 and the receptacle 93 are completely connected, fitting between the metal outer cylinder 143 and the leading end cylinder part 191c is first started. The metal outer cylinder 143 and the leading end cylinder part 191c, which are in an initial fitting state, have an initial tolerance of 400 µm in a radial direction. Further, after the metal outer cylinder 143 and the leading end cylinder part 191c are fitted to each other, the guide key 173 begins to enter the key groove 195. The key groove 195 has a tapered surface and has an initial tolerance of 300 µm on one side thereof at the entrance beginning position.

A cylindrical insulation pressing part 197 is fixed on an inner circumference of the metal housing 191 and fixes an insulating plate 199. The insulating plate 199 is formed with four freely fitting holes 201. In the respective freely fitting holes 201, receptacle-side holders 107 are disposed at positions corresponding to the plug-side holders 105 shown in Fig. 3. Gaps are formed between the freely fitting holes 201 of the insulating plate 199 and outer circumferences of the receptacle-side holders 106. The receptacle-side holders 107 are elastically supported in the plug insertion direction by holder alignment units (which will be described later in detail) so that they can parallel move in a direction orthogonal to the plug insertion direction a. In other words, the annular gaps formed between the freely fitting holes 201 and the receptacle-side holders 107 serve as spaces for movement which allows the receptacle-side holders 107 to move when a core alignment operation is performed.

In the endoscope apparatus 100, plural sets of the plug-side holder 105 and the receptacle-side holder 107 are provided in the optical connector 101 (refer to Fig. 1), and connection/disconnection between the plug-side optical fibers 55 and the receptacle-side optical fibers can be performed at a time. Also, the optical connector 101 itself has an alignment function (not shown). Thus, when the plug 95 is inserted into the receptacle 93, the optical connector 101 cooperates with the holder alignment units (which will be described later) to automatically align the respective receptacle-side holders 105 in an arbitrary direction, thereby collectively performing the optical connection.

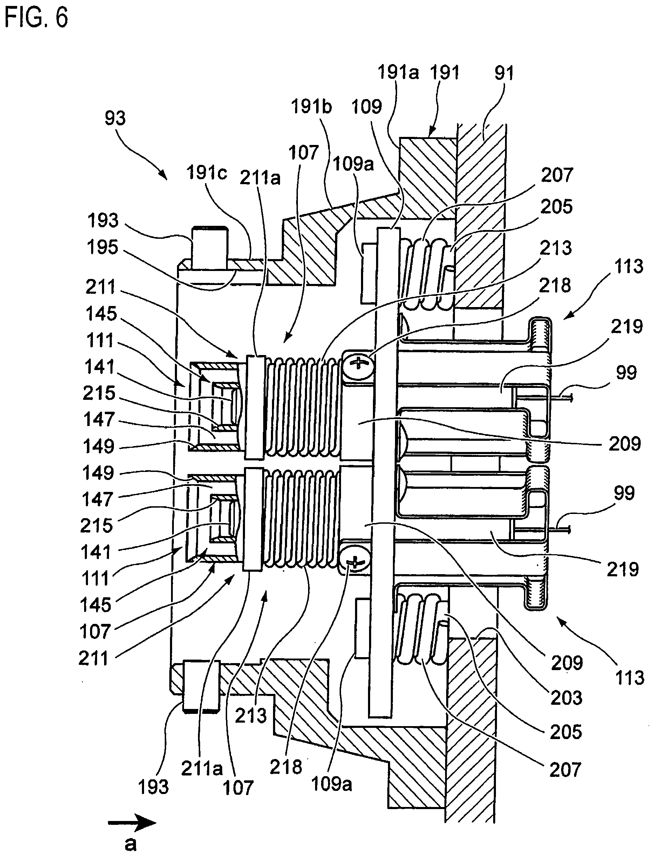

Fig. 6 is a section view taken along an axis line of the receptacle 93. In Fig. 6, the insulation pressing part 197 and the insulating plate 199 are omitted for simplification.

The equipment main body 91 is formed with a through-hole 203 for receptacle. A plurality of support shafts 205 (three support shafts in this illustrative embodiment) protrude at a circumferentially equal distance around the through-hole 203 for receptacle. The support shafts 205 penetrate a disc-shaped floating base 109 so that the floating base 109 can slide, and a larger head part 109a prevents the floating base 109 from detaching therefrom. Coil springs 207 are inserted around the support shafts 205 between the equipment main body 91 and the floating base 109. The coil springs 207 bias the floating base 109 in a direction getting away from the equipment main body 91. When the floating base 109 is pressed in the plug insertion direction a, the floating base 109 can be retreated against the biasing force of the coil springs 207. In other words, the floating base 109 elastically supports the receptacle-side holders 107 in the insertion direction a of the plug 95. The receptacle 93 will be described with the plug insertion direction a referred to as rear and with the direction opposite to the plug insertion direction referred as front.

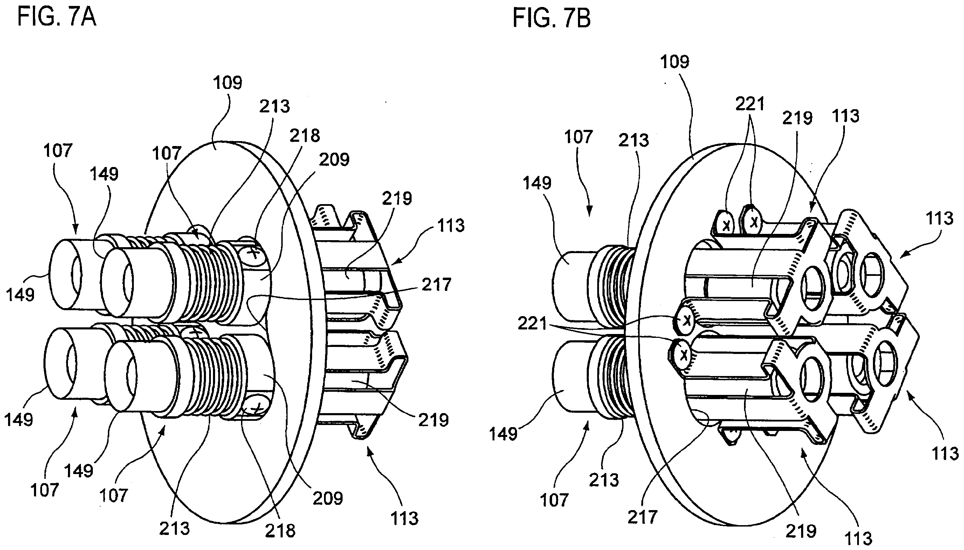

Here, Fig. 7A is a perspective view of the floating base 109 having receptacle-side holders 107 attached thereto when viewed from a diagonally front side thereof. Fig. 7B is a perspective view of the floating base 109 when viewed from a diagonally rear side thereof.

As shown in Figs. 6, 7A and 7B, rear end portions of the four receptacle-side holders 107 are inserted into the through-hole 203, for receptacle, of the equipment main body 91. Large-diameter fixing parts 209 are formed at approximately center portions of the receptacle-side holders 107 in the axial direction thereof. The large-diameter fixing parts 209 are arranged in through hole 217, for holders, of the floating base 109 with a predetermined gap therebetween. Cylindrical socket hoods 211 each having a large-diameter spring seat 211a at outer circumferences thereof are axially inserted to front-side parts of the receptacle-side holders 107. Coil springs 213 are inserted around the receptacle-side holders 107 between the large-diameter spring seats 211a and the large-diameter fixing parts 209. The coil springs 213 bias the socket hoods 211 in the forward direction. The socket hoods 211 are restrained from being forwardly detached from the receptacle-side holders 107 by detachment restraint sections (not shown). With this configuration, when the socket hoods 211 are pressed in the plug insertion direction a, the socket hoods 211 can be retreated against the biasing force of the coil springs 213.

In addition, as shown in Figs. 7A and 7B, the floating base 109 is formed with the through-hole 217 for holders which is formed by connecting circular openings for freely fitting the four receptacle-side holders 107 to each other. Each of the receptacle-side holders 107 is attached with a holder alignment unit 113 having a fixing end supported by the floating base 109. In other words, the holder alignment units 113 are fixed to the large diameter parts 209 by fixing screws 218, respectively. The holder alignment units 113 fixed to the receptacle-side holders 107 are inserted into the through-hole 217 for holders and then fixed to the floating base 109 by fixing screws 221.

The holder alignment units 113 are provided between the receptacle-side holders 107 and the floating base 109. Thereby, the receptacle-side holders 107 can be moved with respect to the floating base 109 in a direction perpendicular to the insertion direction a of the plug 95. With this configuration, the receptacle-side holders 107 are supported so that the optical axes of receptacle-side optical fibers 99 and the optical axes of the plug-side optical fibers 55 can be aligned. In other words, when the plug-side optical fibers 55, which is fixed to the plug 95, and the receptacle-side optical fibers 99, which is fixed to the receptacle 93, are optically connected by detachably mounting the plug 95 to the receptacle 93, which is provided in the equipment main body 91, the holder alignment units 113 support the receptacle-side holders 107 so that the receptacle-side holders 107 can parallel move in a direction perpendicular to a connector connection direction, thereby aligning the optical axes.

Here, the specific configuration of the holder alignment units 113 will be described.

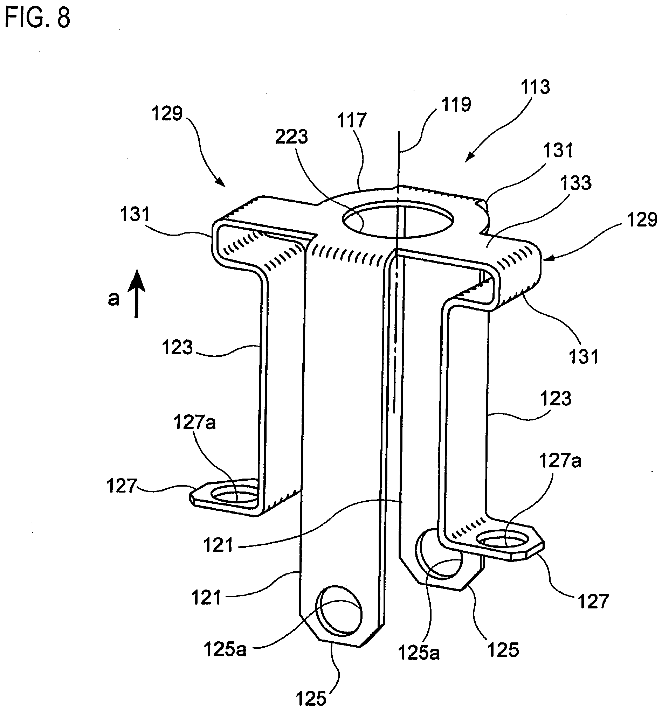

Fig. 8 is a perspective view of the holder alignment units 113 shown in Fig. 7.

The holder alignment unit 113 has a common plate part 117, a pair of first parallel linear support pieces 121, 121 and a pair of second parallel linear support pieces 123, 123. The pair of first parallel linear support pieces 121, 121 vertically protrude from the common plate part 117 with a virtual axis 119 perpendicular to the common plate part 117 being interposed therebetween. The pair of second parallel linear support pieces 123, 123 vertically protrude from the common plate part 117 at positions which are rotated about the virtual axis 119 by 90° from the first linear support pieces 121, 121.

In the holder alignment unit 113, first leading end portions 125 of the first linear support pieces 121 are fixed to the receptacle-side holder 107, and second leading end portions 127 of the second linear support pieces 123 are fixed to the floating base 109. The first leading end portions 125 are formed with fixing holes 125a through which the fixing screws 218 penetrate. The second leading end portions 127 are formed with fixing holes 127a through which the fixing screws 221 penetrate. Also, the common plate part 117 is formed with an opening 223 through which the receptacle-side optical fiber 99 penetrates.

The first linear support pieces 121 and the second linear support pieces 123 respectively protrude in the same direction from the common plate part 117. With this configuration, it is possible to suppress the protrusion heights of the first linear support pieces 121 and second linear support pieces 123 from the common plate part 117, thereby improving space efficiency for arrangement.

Also, spring parts 131 that are elastically deformable in the insertion direction a of the plug 95 are formed in connection portions 129 of the common plate part 117 where the common plate part 117 is connected to the second linear support pieces 123. The spring parts 131 are formed by folding the second linear support pieces 123 formed of a band-shaped plate into a U shape. Since the holder alignment units 113 are elastically deformed in the insertion direction a of the plug 95 by the spring parts 131, a close contact property between connection end portions of an optical connection end face part of the plug-side ferrule 135 and an optical connection end face part of a receptacle-side ferrule 139 can be improved. Also, the spring parts 131 are deformed even when the receptacle-side holder 107 parallel moves, so that it enables the receptacle-side holder 107 to parallel move easily.

In the meantime, in the holder alignment unit 113, at least the first linear support pieces 121 and the second linear support pieces 123 are formed of a plate member 133, so that the holder alignment units 113 can more surely parallel displace the receptacle-side holder 107 when the respective support pieces 121, 123 are elastically deformed. Also, each holder alignment unit 113 may be formed of a single metal plate. In this case, it is possible to simply manufacture the holder alignment unit 113 with high precision by a press molding and to thus minimize the number of parts.

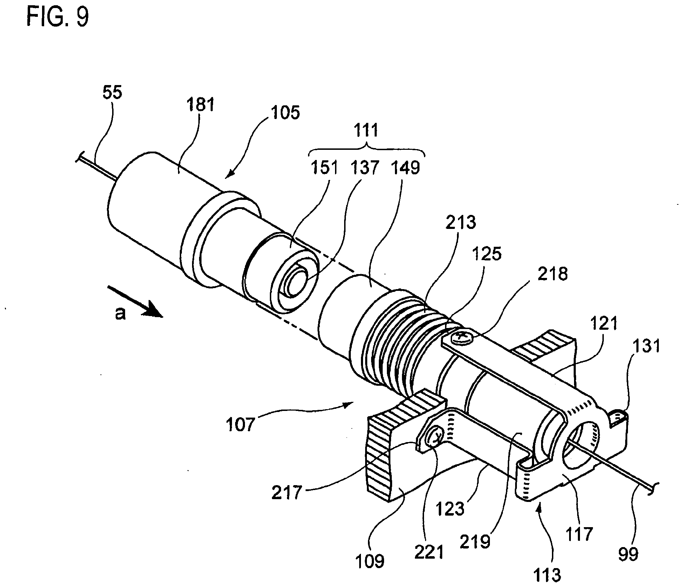

Fig. 9 is a perspective view of the plug-side holder 105 and the receptacle-side holder 107 just before connection.

The holder alignment unit 113 enables, by the first linear support pieces 121 and the second linear support pieces 123, the receptacle-side holder 107 to move in the direction perpendicular to the plug insertion direction a while keeping the receptacle-side holder 107 being parallel to the floating base 109. In this manner, in connecting the receptacle 93 and the plug 95, when a relative position between the receptacle-side holder 107 and the plug-side holder 105 is regulated by an engagement unit 111, the receptacle-side holder 107 is displaced relatively to the plug-side holder 105 in the direction perpendicular to the insertion direction a of the plug 95, and the optical axis of the receptacle-side optical fiber 99 is aligned to the optical axis of the plug-side optical fiber 55. At this time, the coil spring 213 serves as a cushion in the insertion direction a.

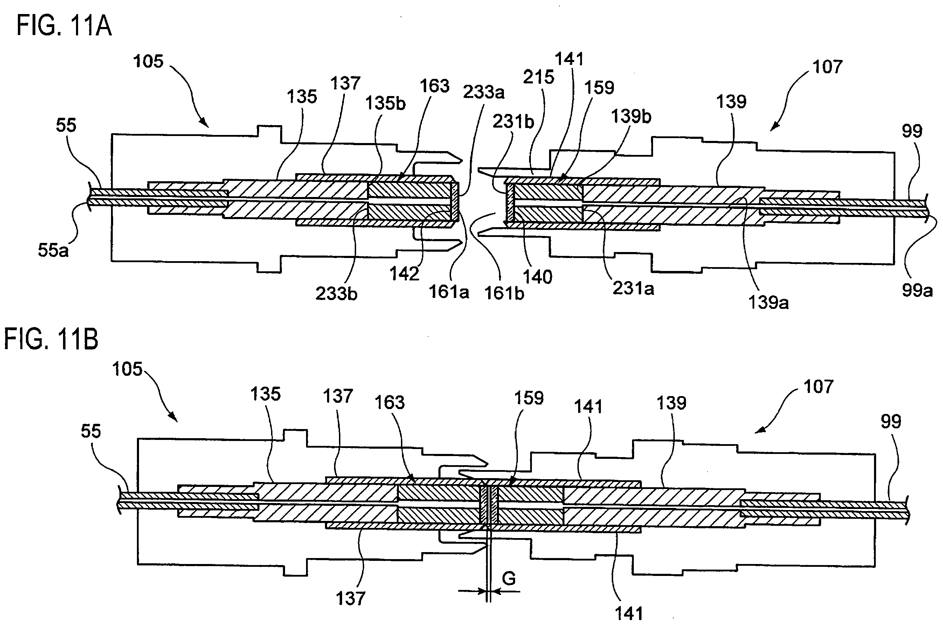

Here, the optical axes of the plug-side holder 105 and the receptacle-side holder 107 are shown in Figs. 11A and 11B. Fig. 11A is a section view showing a connection configuration of optical fibers before connection. Fig. 11B is a section view showing the connection configuration of optical fibers after the connection.

The receptacle-side holder 107 has a gap in the socket hood 211 (refer to Fig. 6) and coaxially fixes an outer sleeve 215. Also, a receptacle-side inner sleeve 141 is coaxially housed in the outer sleeve 215 so that it is slidable. A rear part of the receptacle-side inner sleeve 141 is inserted into a pressing cylinder 219 (refer to Figs. 7 and 9) fixed to the rear part of the receptacle-side holder 107. In rear of the pressing cylinder 219, a coil spring (not shown) is housed. The coil spring biases the receptacle-side inner sleeve 141 in the forward direction. When the receptacle-side inner sleeve 141 is pressed in the plug insertion direction a, the receptacle-side inner sleeve 141 can be retreated against the biasing force of the coil spring.

The receptacle-side inner sleeve 141 is a member that covers the receptacle-side ferrule 139 which will be described in detail later. The plug-side inner sleeve 137 is a member that covers the outer circumference of the plug-side ferrule 135 fixing the plug-side optical fiber 55.

As shown in Fig. 6, the outer circumference of the receptacle-side inner sleeve 141 is covered by the outer sleeve 215. In connecting the connectors, the outer sleeve 215 serves as a protrusion 145 that is engaged with the outer circumference of the plug-side inner sleeve 137 shown in Fig. 4. In other words, the protrusion 145, which is the receptacle-side outer sleeve 215, and the plug-side inner sleeve 137, which serves as an engagement part to be engaged with the protrusion 145, constitute an engagement pair, and they are engaged with each other in connecting the optical connectors, so that they are positioned with high precision. In this illustrative embodiment, the protrusion 145 is provided at the leading end of the outer sleeve 215, which is fitted onto the outer circumference of the receptacle-side inner sleeve 141. However, the protrusion 145 may be provided on the outer circumference of the plug-side inner sleeve 137 shown in Fig. 4.

Also, a front part of the socket hood 211 is formed with a receptacle-side cylinder part 149 that is to be engaged with a plug-side cylinder part 151 which is provided in the leading end of the plug-side holder 105 shown in Fig. 4. In other words, when the plug-side holder 105 is inserted into the receptacle-side holder 107, an inner circumference of the receptacle-side cylinder part 149, which serves a protrusion formed in the socket hood 211 of the receptacle-side holder 107, and an outer circumference of the plug-side cylinder part 151 of the plug-side holder 105, which serves an engagement part, are engaged with each other, so that it is possible to match the insertion positions of the receptacle 93 and plug 95. An axial deviation of the plug 95 in the direction perpendicular to the insertion direction a, which occurs in the engagement operation, is absorbed by the holder alignment units 113.

The respective leading ends of the socket hood 211, the outer sleeve 215 and the receptacle-side inner sleeve 141 are arranged at positions which are retreated from the leading end cylinder part 191c in this order. In the plug-side holder 105, the respective leading ends of the plug-side holder 105 and the plug-side inner sleeve 137 are arranged in positions which are retreated from the leading end of the metal outer cylinder 143 in this order.

In this manner, in the optical connector 101 having the above configuration, the protrusion 145, the receptacle-side cylinder part 149 and the plug-side cylinder part 151 are arranged so that a position at which the protrusion 145 begins to engage with the plug-side inner sleeve 137 and a position at which the receptacle-side cylinder part 149 begins to engage with the plug-side cylinder part 151 are different in terms of the insertion direction a of the plug 95. Also, the protrusion 145, the receptacle-side cylinder part 149 and the plug-side cylinder part 151 are configured to have the gaps, in the direction perpendicular to the insertion direction, with the respective engagement counterparts so that (i) one which earlier begins to engage with its counterpart than another one as the plug 95 is inserted has a wider gap and (ii) one which later begins to engage with its counterpart than another one as the plug 95 is inserted has a narrower gap.

Also, when the metal outer cylinder 143 of the plug 95 shown in Fig. 4 is inserted into the inner circumference of the metal housing 191 of the receptacle 93 shown in Fig. 6, the above is also the same for an engagement between the leading end cylinder part 191c and the metal outer cylinder 143 and an engagement between the guide key 173 provided in the metal outer cylinder 143 and the key groove 195 of the leading end cylinder part 191c. In other words, the leading end cylinder part 191c and the metal outer cylinder 143, which are the earliest engaged, are arranged so that they most protrude in the plug insertion direction, and the guide key 173 provided in the metal outer cylinder 143 is arranged at the next protruding position. The gaps in the direction perpendicular to the insertion direction are configured so that the gap between the elements which later engage with each other is narrower than the gap between the elements which earlier engage with each other.

Thereby, as the plug 95 is inserted, the positioning precision is enhanced in a stepwise fashion. In other words, by only inserting the plug 95 into the receptacle 93, the positioning precision is automatically secured at the final connection state. Also, the optical connector including the optical parts such as the optical fibers is configured so that the respective engagement pairs are engaged in a stepwise manner at different timings. Thereby, it is possible to prevent shock, which may be caused the insertion operation of the plug 95, from being strongly transmitted to the parts (near the optical axis) that are connected with the highest precision. Accordingly, it is possible to improve shock-resistance of the optical connector and to provide the optical connector having an excellent handling property.

Furthermore, the optical connector 101 having the above configuration is further provided with the plurality of engagement pairs, such as the receptacle-side cylinder part 149 and the plug-side cylinder part 151, so that the positions where ones of the respective pairs begin to engage with the others of the respective pairs are different. Thereby, it is possible to realize the higher positioning precision.

Also, the connection operation between the receptacle 93 and the plug 95 is performed through different insertion operations of two stages, i.e., a first engagement operation between the leading end cylinder part 191c of the receptacle 93 and the metal outer cylinder 143 of the plug 95 and a second engagement operation between the plug-side holders 105 and the receptacle-side holders 107.

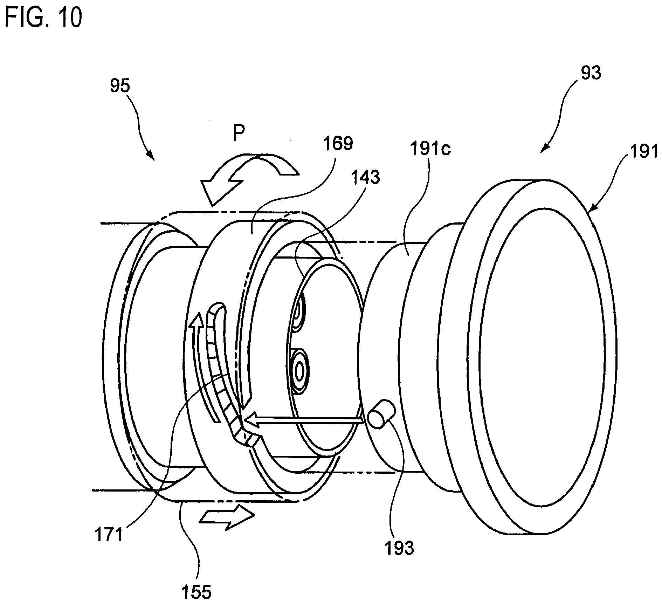

Here, Fig. 10 is a perspective view of the plug 95 and the receptacle 95 showing engagement between the cam cylinder 169 and the engagement pins 193. As shown in Fig. 10, when the ring handle 155 is rotated with the engagement pins 193 being in contact with the leading end face of the cam cylinder 169, the engagement pins 193 are received in the guide grooves 171 at a rotation position where entrances of the guide grooves 171 formed in the cam cylinder 169 are matched to the engagement pins 193. Then, when the ring handle 155 is rotated in an arrow direction P, the engagement pins 193 are guided along the guide grooves 171, so that the receptacle 93 and the plug 95 are connected. In other words, in the second engagement operation, when the ring handle 155 is rotated, the engagement pins 193 provided at the leading end cylinder part 191c of the receptacle 93 are engaged with the cam cylinder 169 of the plug 95, so that the axial insertion speed is reduced. Thereby, it is possible to connect the plug-side holders 105 and the receptacle-side holders 107 without applying shock, which may occur in performing the connector connection, to the holders 105, 107.

The guide grooves 171, the engagement pins 193, the guide key 173, the key groove 195, the protrusion 145, the plug-side inner sleeves 137, the receptacle-side cylinder parts 149, the plug-side cylinder parts 151, the leading end cylinder part 191c and the metal outer cylinder 143 constitute the engagement unit 111, which engages the plug 95 and the receptacle 93 with high precision.

### <Optical Connection in Respective Holders>

Next, an optical connection configuration between the receptacle-side holder 107, which is supported by the holder alignment units 113, and the plug-side holder 105 will be described.

As shown in Figs. 11A and 11B, in the optical connector 101 having the above configuration, the plug-side holder 105 and the receptacle-side holder 107 are connected to optically connect the plug-side optical fibers 55 and the receptacle-side optical fibers 99. In the plug-side holder 105, the outer circumference of the plug-side ferrule 135 fixing the plug-side optical fiber 55 is covered by the plug-side inner sleeve 137. In the receptacle-side holder 107, the outer circumference of the receptacle-side ferrule 139 fixing the receptacle-side optical fiber 99 is covered by the receptacle-side inner sleeve 141. In the optical connector 101 having the above configuration, the optical axes are matched with high precision by the engagement units 111 and the holder alignment units 113. Also, the optical connector further includes an end face connection structure for securing a low-loss connection even when the optical axes are slightly deviated.

Specifically, in the receptacle-side holder 107, a first graded index collimator (first GI collimator) 159 is incorporated in an optical connection end face part 161b of the receptacle-side ferrule 139. The first GI collimator 159 enlarges and collimates a beam diameter of light which is incident from the receptacle-side optical fiber 99. Also, in the plug-side holder 105, a second graded index collimator (second GI collimator) 163 is incorporated in an optical connection end face part 161a of the plug-side ferrule 135. The second GI collimator 163 has the approximately same diameter as that of the first GI collimator 159, converges the beam diameter of the light which is incident from the first GI collimator 159, and inputs the light into the plug-side optical fiber 55.

The first GI collimator 159 and the receptacle-side ferrule 139 are detachably connected by the receptacle-side inner sleeve 141. Also, the second GI collimator 163 and the plug-side ferrule 135 are detachably connected by the plug-side inner sleeve 137. The receptacle-side inner sleeve 141 and the plug-side inner sleeve 137 may be made of any of various materials, such as a metal or zirconium oxide ceramic.

The receptacle-side ferrule 139 has a cylindrical shape and is formed with a fiber insertion opening 139a, which axially penetrates, at its center. The receptacle-side optical fiber 99 whose sheath 99a is peeled off at its leading end is inserted into the fiber insertion opening 139a and fixed by an adhesive. It is noted that although Figs. 11A and 11B show the simplified configuration of the illustrative embodiment of the invention, in practice, a metal flange adhered to a base side of the receptacle-side ferrule 139 maintains the receptacle-side optical fiber 99 in connecting the receptacle-side optical fiber 99 and the receptacle-side ferrule 139. A leading end 139b of the receptacle-side ferrule 139 and a leading end of the receptacle-side optical fiber 99 inserted into the fiber insertion opening 139a are polished into a convex spherical shape or a planar shape together.

An incident end face 231a and an emitting end face 231b of the first GI collimator 159 are polished into a convex spherical shape and a planar shape, respectively. Also, the incident end face 231a of the first GI collimator 159 contacts with the leading end 139b of the receptacle-side ferrule 139, so that it is physically connected to the receptacle-side optical fiber 99. A seal glass 140 is provided on the emitting end face 231b.

The first GI collimator 159 enlarges and collimates the beam diameter of the laser light transmitted by the receptacle-side optical fiber 99. Therefore, a light power density at the emitting end face 231b is lower than that at the leading end of the receptacle-side optical fiber 99, and the connection loss is prevented from being lowered due to foreign substances, scratch and the like of the emitting end face 231b.

The second GI collimator 163 has the substantially same configuration as the first GI collimator 159, and converges and introduces the light having the beam diameter enlarged by the first GI collimator 159 into the plug-side optical fiber 55. Also, the second GI collimator is held in the plug-side inner sleeve 137 together with the plug-side ferrule 135, and an incident end face 233a and an emitting end face 233b of the second GI collimator are polished into a planar shape and a convex spherical shape, respectively. A seal glass 142 is provided on the incident end face 233a.

Also, the seal glass 142 of the incident end face 233a faces the receptacle-side seal glass 140 with a predetermined gap G. The emitting end face 233b of the second GI collimator 163 contacts with the leading end of the plug-side ferrule 135, so that it is physically connected to the plug-side optical fiber 55.

The plug-side ferrule 135 is the same part as the receptacle-side ferrule 139 and holds the leading end of the plug-side optical fiber 55, like the receptacle-side ferrule 139. The leading end 135b of the plug-side ferrule 135 and the leading end of the plug-side optical fiber 55 is polished into a convex spherical shape (or planar shape) together.

When the receptacle-side holder 107 and the plug-side holder 105 are fitted, the receptacle-side inner sleeve 141 comes into contact with the plug-side inner sleeve 137. The seal glass 140 is retreated from the end of the receptacle-side inner sleeve 141. The seal glass 142 is protruded from the end of the plug-side inner sleeve 137. Thus, the sleeves 137, 141 first come into contact with each other before the seal glass 142 is in contact with the seal glass 140 when the holders 105, 107 are fitted to each other. Thereby, the predetermined gap G is formed therebetween.

In general, it has been known that foreign substance which may be attached to a leading end of an optical fiber has a size of 50 µm at maximum. It is, therefore, necessary that the predetermined distance G should be 50 µm or larger so as to prevent the attached foreign substance from being sandwiched and crushed therebetween. Further, the gap G is preferably about 1.0 mm to 2.0 mm, considering the manufacturing error, the assembly error and the like of the respective constitutional parts of the end face structure.

### <Operations in Connecting Connector>

Next, operations in connecting the optical connector 101 will be described.

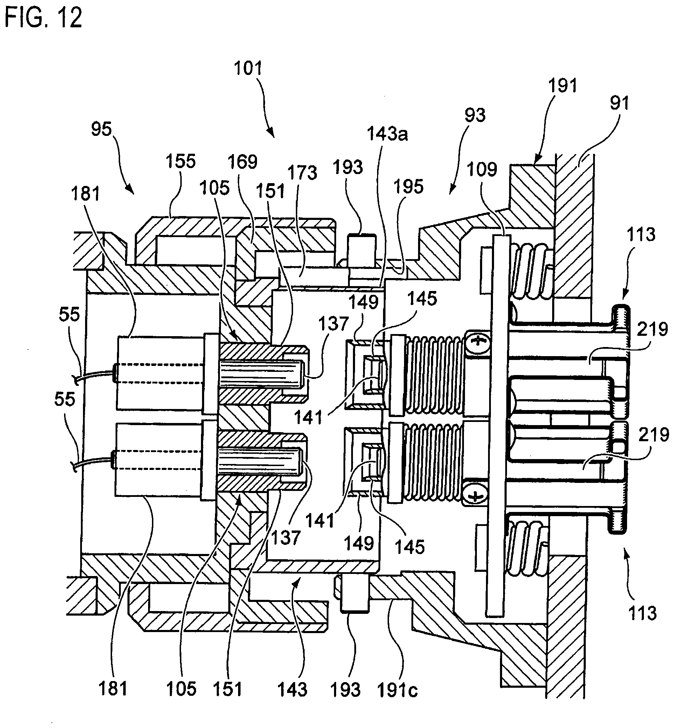

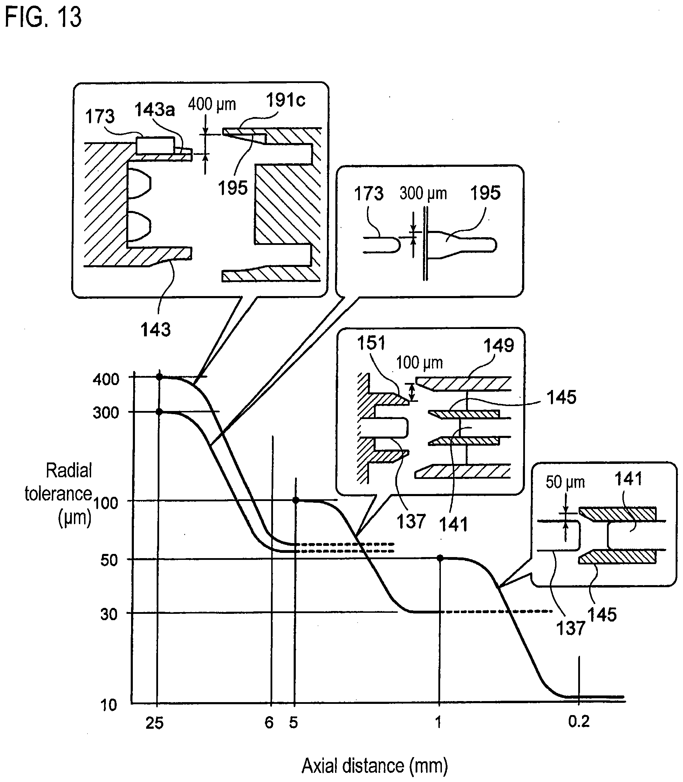

Fig. 12 is a section view of the plug 95 and the receptacle 93 before connection beginning. Fig. 13 is a graph showing a relation between an axial distance and a radial tolerance in each engagement portion together with a schematic view of main parts.

As shown in Fig. 12, at first, the connection of the optical connector 101 begins with inserting of the metal outer cylinder 143 of the plug 95 into the leading end cylinder part 191c of the metal housing 191 of the receptacle 93. The guide key 173 provided in the metal outer cylinder 143 is matched to and inserted into the key groove 195 of the leading end cylinder part 191c. At this time, an initial tolerance between a key groove 143a, in which the guide key 173 is disposed, and the key groove 195 of the leading end cylinder part 191c is set to be 400 µm, as shown in Fig. 13. Also, the key groove 195 is wider on its insertion side. A gap, in a rotating direction, between the key groove 195 and the guide key 173 is about 300 µm on each side. When the plug 95 is inserted while fitting the guide key 173 and the key groove 195 to each other, a radial tolerance becomes about 70 µm at a time at which an axial distance therebetween extending the gap G in the insertion direction becomes about 6 mm.

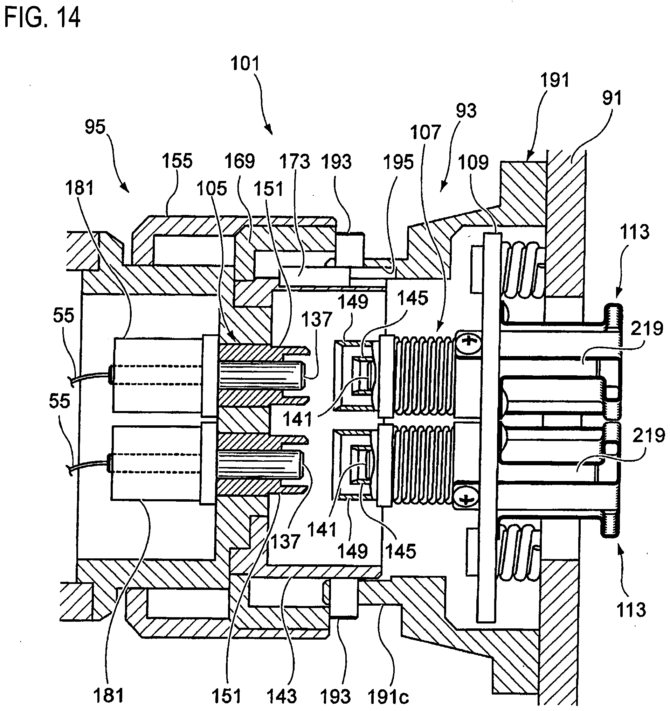

From the state of Fig. 12, when the plug 95 is continuously inserted, the plug 95 is in a state shown in Fig. 14. Fig. 14 is a section view of the plug 95 with which the engagement pins 193 are in contact and the receptacle 93.

When the metal outer cylinder 143 is inserted into the leading end cylinder part 191c, the leading end face of the cam cylinder 169 comes into contact with the engagement pins 193, 193 provided in the upper and lower portions of the leading end cylinder part 191c. Thereby, the insertion of the plug 95 is stopped. In a state where the leading end face of the cam cylinder 169 is in contact with the engagement pins 193, the receptacle-side cylinder parts 149 which are the leading ends of the receptacle-side holders 107 are spaced from the plug-side cylinder parts 151 which are the leading ends of the plug-side holders 105.

As shown in Fig. 10, when the ring handle 155 is further rotated in the state where the engagement pins 193 are introduced in the guide grooves 171, the engagement pins 193 are in sliding contact with the inclined guide grooves 171, so that the plug 95 approaches the engagement pins 193.

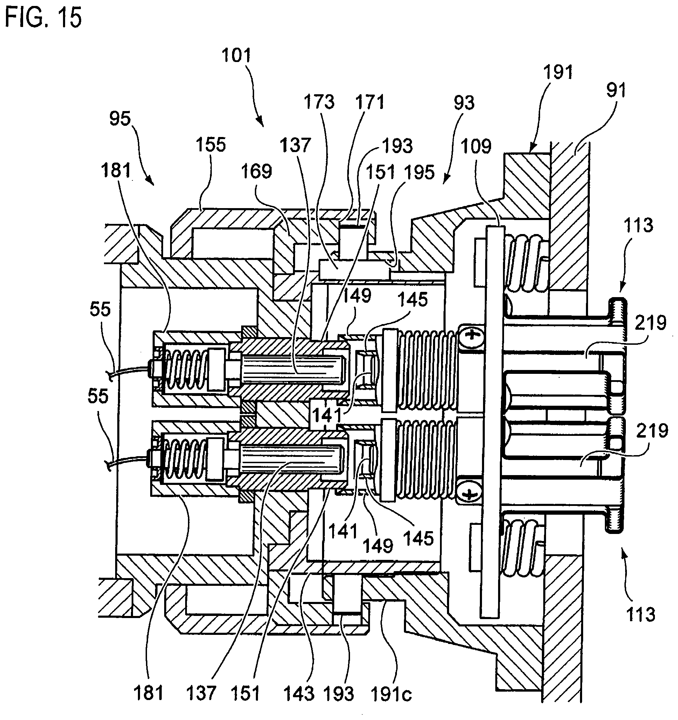

As the approach of the plug 95, as shown in Fig. 15, the plug-side cylinder parts 151 begin to engage in the receptacle-side cylinder parts 149. Although the receptacle-side cylinder parts 149 and the plug-side cylinder parts 151 have the initial tolerance of 100 µm as shown in Fig. 13, the tapered surfaces of them first come into contact with each other as the plug 95 is inserted. Here, when an axis deviation occurs between them, a reaction force from the plug-side cylinder parts 151 acts on the receptacle-side cylinder parts 149. The reaction force is directed to any direction orthogonal to the central axes of the receptacle-side holders 107 and is transmitted to the holder alignment units 113, and the optical axes are thus aligned.

### <Core Alignment Operation of Holder Alignment Units>

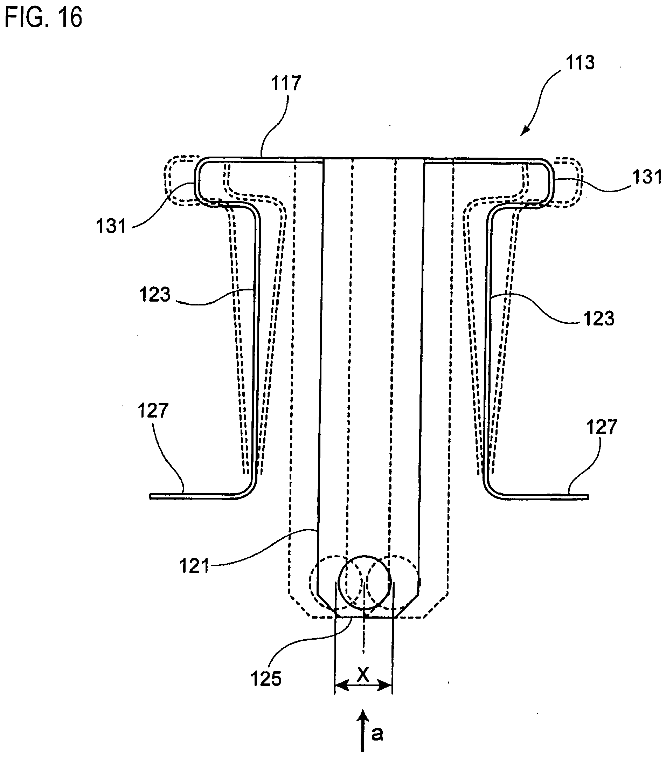

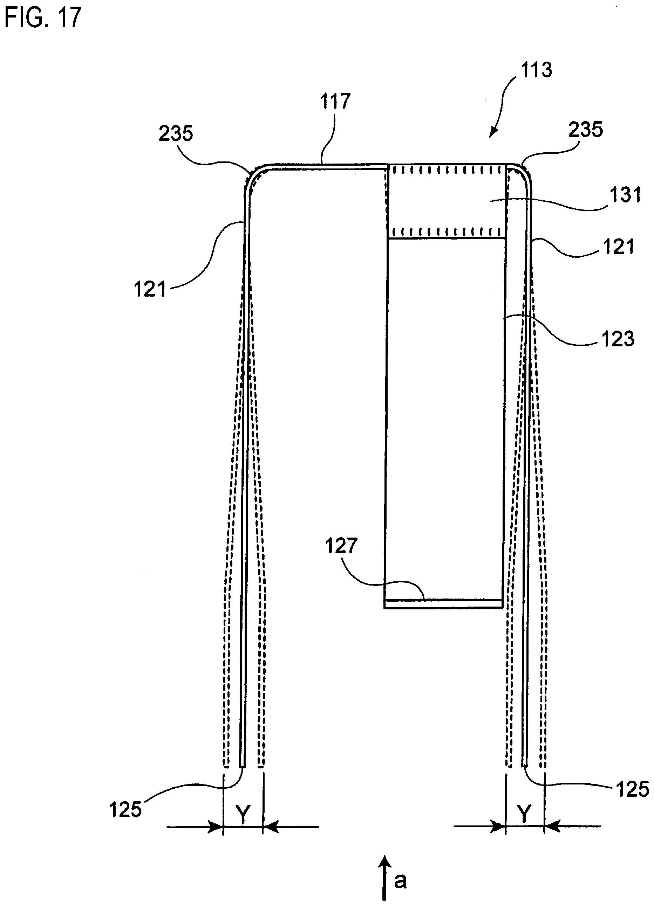

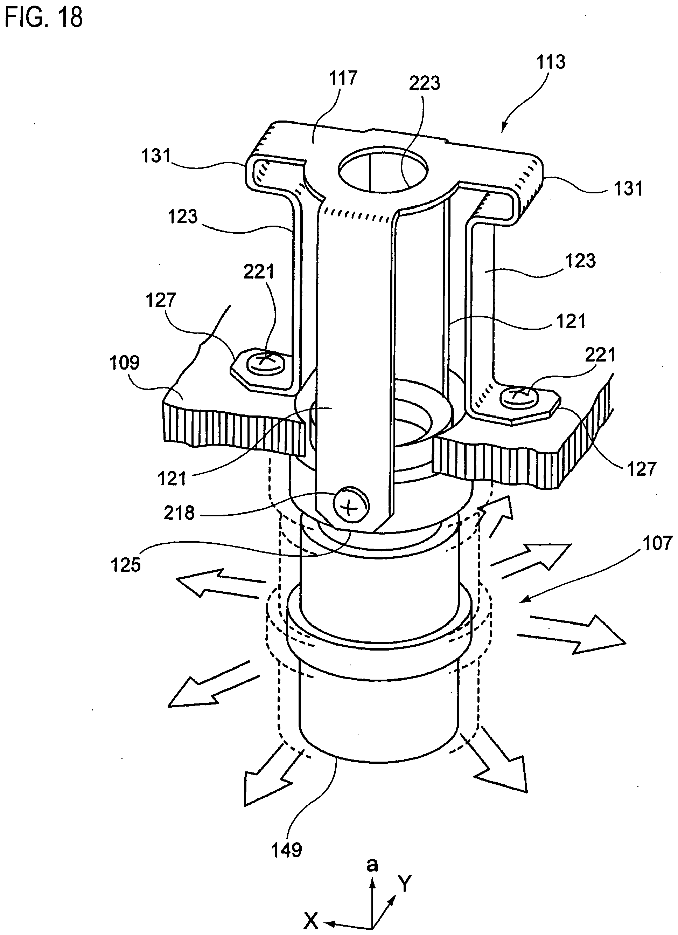

Fig. 16 illustrates a core alignment operation by displacement of the second linear support pieces 123. Fig. 17 illustrates a core alignment operation by displacement of the first linear support pieces 121. Fig. 18 is a schematic view of the holder alignment unit 113 illustrating a core alignment operation by displacement of the first and second linear support pieces 121, 123.

As shown in Fig. 16, when the reaction force orthogonal to the axis core acts on the receptacle-side holder 107, the receptacle-side holder 107 tries to move in the through-hole 217 for holder of the floating base 109. This motion is allowed by deformation of the holder alignment unit 113. When the reaction force from the receptacle-side holder 107 acts on the first linear support pieces 121 and the direction of the reaction force is a direction away from the second linear support pieces 123, 123 (left-right direction in Fig. 16), the holder alignment unit 113 obliquely deform the second linear support pieces 123, 123 in parallel, which have the leading end portions 127 fixed to the floating base 109. In other words, the holder alignment unit 113 is deformed while maintaining its parallelogram shape. Accordingly, the common plate part 117 keeps the second leading end portions 127 being parallel to each other, and the receptacle-side holder 107 fixed to the first linear support pieces 121, 121 can parallel move in a direction (X direction) orthogonal to the plug insertion direction a.

Next, it is assumed that the direction of the reaction force from the receptacle-side holder 107 is a direction (left-right direction in Fig. 17) away from the first linear support pieces 121, 121 as shown in Fig. 17. In this case, since the second leading end portions 127 of the second linear support pieces 123 are fixed to the floating base 109, the first linear support pieces 121, 121 are deformed to have a moderate S shape or a reverse S shape with the connection portions 235, 235 between the common plate part 117 and the first linear support pieces 121, 121 serving as base points. In other words, the first leading end portions 125, 125 of the first linear support pieces 121, 121 can displace in the direction (left-right direction in Fig. 17) away from the first linear support pieces 121, 121. Therefore, the receptacle-side holder 107 fixed to the first linear support pieces 121, 121 can parallel move in a direction (Y direction) orthogonal to the plug insertion direction a.

In this manner, the holder alignment unit 113 supports the receptacle-side holder 107 so that the receptacle-side holder 107 can move in the XY directions as shown in Fig. 18. Thereby, the receptacle-side holder 107 can parallel move in any direction on the XY plane orthogonal to the plug insertion direction a.

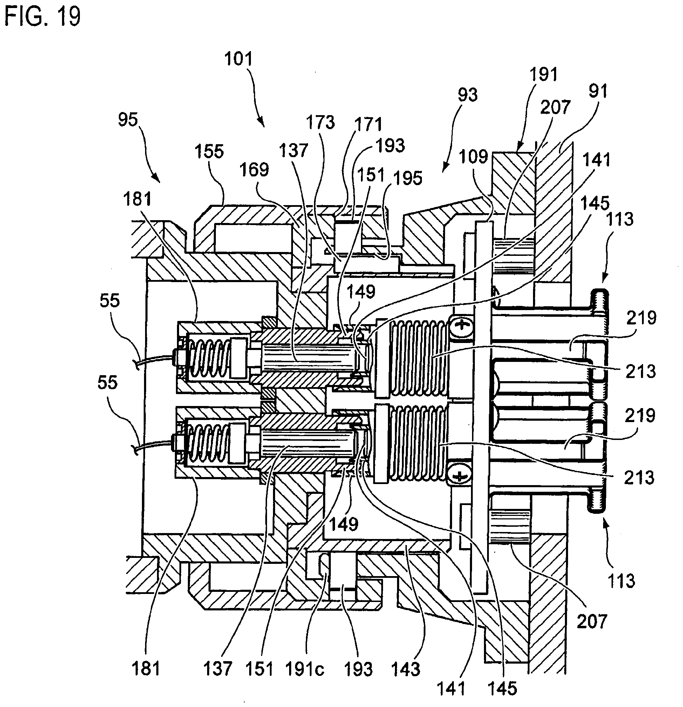

Fig. 19 is a section view of the plug 95 and the receptacle 93 after the engagement pins 193 are inserted and the ring handle 155 is rotated.

The approach of the plug 95 is stopped at a position at which the ring handle 155 cannot be further rotated, i.e., a position at which the engagement pins 193 reach terminals of the guide grooves 171. During the rotating operation of the ring handle 155, the receptacle-side cylinder parts 151 and the plug-side cylinder parts 149 engage with each other and then, the protrusions 145 and the plug-side inner sleeves 137 engage with each other. As shown in Fig. 13, the initial tolerance between the plug-side inner sleeve 137 and the protrusion 145 of the outer sleeve is about 50 µm. This tolerance is narrowed as the plug 95 is inserted. Thereby, the plug 95 is completely connected to the receptacle 93 with the axes being matched with high precision.

In connecting the plug 95 and the receptacle 93, the position deviation from the plug insertion direction a is absorbed by the coil springs 207, 123, 183 and the spring parts 131, and the optical connection end face part 161a and the optical connection end face part 161b are positioned to have the predetermined spaced distance G.

As shown in Fig. 13, at the position which is 25 mm before the completion of connection, the radial tolerance between the key groove 143a of the guide key 173 and the key groove 195 is 400 µm, and the tolerance in the rotating direction is 300 µm. At a position which is 5 mm before the completion of connection, the receptacle-side cylinder parts 149 and the plug-side cylinder parts 151 begin to engage with each other. The receptacle-side cylinder part 149 and the plug-side cylinder part 151 having begun the engagement have a radial tolerance of 100 µm. Then, at a position which is 1 mm before the completion of connection, the protrusions 145 and the plug-side inner sleeves 137 begin to engage with each other, and the radial tolerance becomes 50 µm. Furthermore, when the connection is completed, the radial tolerance is suppressed to be 20 µm or smaller.

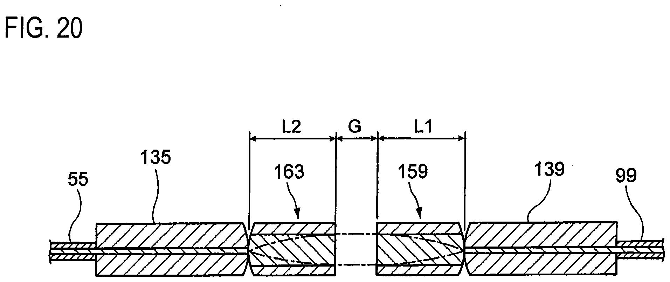

The receptacle 93 and the plug 95, which are aligned by the holder alignment units 113, are surely connected with low loss by the first GI collimators 159 and the second GI collimators 163. Fig. 20 schematically illustrates a light transmission state by the first GI collimator 159 and the second GI collimator 163. It is noted that, in Fig. 20, the gap G is drawn wider in order to clarify the light transmission state between the first GI collimator 159 and the second GI collimator 163. When both an axial length L1 of the first GI collimator 159 and an axial length L2 of the second GI collimator 163 are 1/4 pitch (1 pitch is a length of one period of minimum-maximum-minimum-maximum of a mode field system of light transmitted by a GI collimator), the light having the beam diameter enlarged and collimated by the first GI collimator 159 is incident on the second GI collimator 163 without a great loss and is converged and incident on the plug-side optical fiber 55 by the second GI collimator 163.

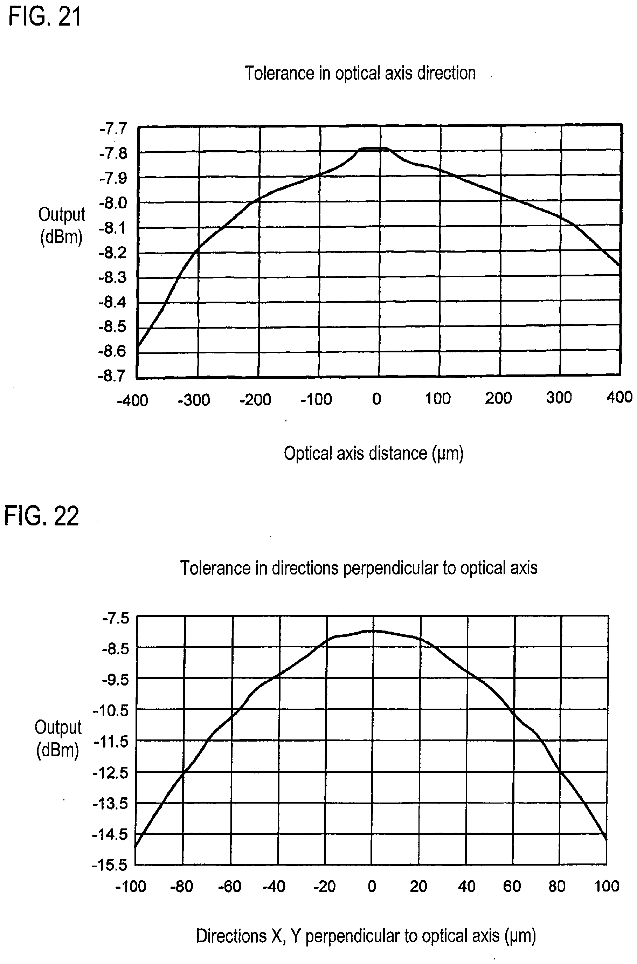

Figs. 21 and 22 show tolerance curves indicating outputs of laser lights that are output from the plug-side optical fibers 55 in the case a relative position between the first GI collimator 159 and the second GI collimator 163 is deviated in an optical axis direction Z and in the case where the relative position is deviated in the directions X, Y orthogonal to the optical axis direction Z, respectively. A plus direction of the optical axis direction Z indicates a direction in which the first GI collimator direction 159 and the second GI collimator 163 approach to each other. Also, the directions X, Y orthogonal to the optical axis shown in Fig. 22 are in a state where deviation in the optical axis direction Z is zero. The tolerance curves are measurement results when lengths of the first GI collimator 159 and the second GI collimator 163 are about 4.0 mm to 4.6 mm, tolerance in the optical axis direction is ±100 µm, and tolerances in the directions X, Y are ±20 µm.

As can be seen from Figs. 21 and 22, an output decrease of the laser light is relatively small with respect to deviation of the relative position between the first GI collimator 159 and the second GI collimator 163. That is, the beam diameter of the light emitted from the first GI collimator 159 is enlarged to thereby improve a margin of the positioning precision of the plug 95 and the receptacle 93. Therefore, the end face structure of the optical connector 101 can maintain the low-loss characteristic even when a deviation occurs in the relative position between the first GI collimator 159 and the second GI collimator 163.

In this manner, the optical connector 101 having this configuration can increase the positioning precision step by step as the plug 95 is inserted, so that the low-loss connection can be simply realized.

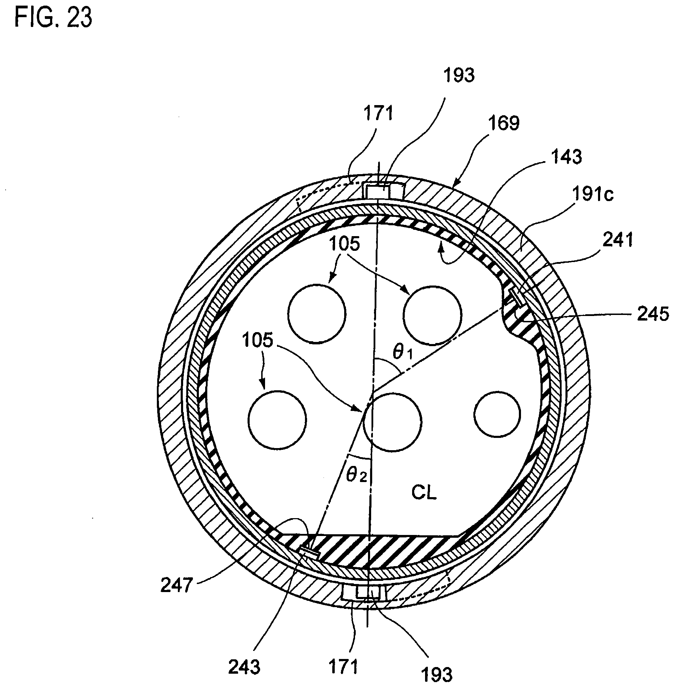

In the above illustrative embodiment, the single guide key 173 is used in the connection structure between the plug 95 and receptacle 93 so as to fit the plug 95 and the receptacle 93. Instead, the connection structure whose section is schematically shown in Fig. 23 may be adopted. In other words, a plurality of inner engagement pins 241, 243, which inwardly protrude from the inner circumference of the leading end cylinder part 191c, are respectively arranged at different circumferential angles θ₁, θ₂ from a center line CL connecting the pair of engagement pins 193 provided in the leading end cylinder part 191c. Also, linear grooves 245, 247 are formed along the plug insertion direction and at peripheral positions, which is located at the circumferential angles θ₁, θ₂ and which correspond to the inner engagement pins 241, 243, on the outer circumference of the metal outer cylinder 143 of the plug 95. With this connection structure, when the engagement pins 193 are inserted in the guide grooves 171 of the cam cylinder 169 of the plug 95, the inner engagement pins 241, 243 of the receptacle 93 are guided and fitted to the linear grooves 245, 247 of the metal outer cylinder 143 of the plug 95. In other words, the metal outer cylinder 143 of the plug 95 is inserted into the leading end cylinder part 191c of the receptacle 93 with the axis cores thereof being matched with high precision by the fitting between the inner engagement pins 241, 243 and the linear recesses 245, 247.

Thereby, when the metal outer cylinder 143 of the plug 95 is engaged with the leading end cylinder part 191c of the receptacle 93, it is possible to match the axis cores thereof with higher precision. Further, it is possible to smoothly continue the insertion operation for a pair of engagement members, which will begin to engage next.

### <Modified Embodiment of Holder Alignment Unit>

Next, the holder alignment unit according to modified embodiments will be described.

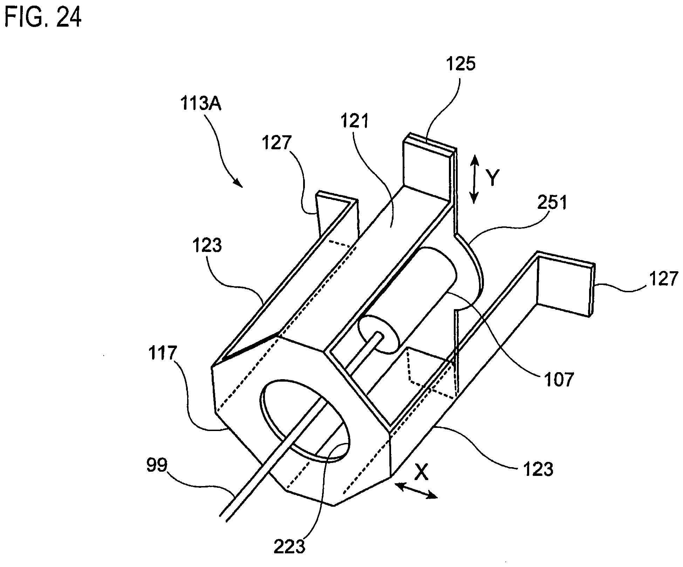

Fig. 24 is a perspective view of a holder alignment unit according to one modified embodiment in which a receptacle-side holder 107 is fixed to first linear support pieces 121 via a vertical support plate 251.

In a holder alignment unit 113A of this modified embodiment, a central portion of the support plate 251 is fixed to the leading end face of the receptacle-side holder 107, and both ends of the support plate 251 are fixed to the first leading end portions 125, 125 of the first linear support pieces 121, 121. Also, no spring parts 131 are formed between the common plate part 117 and the second linear support pieces 123. With this modified embodiment, the holder alignment unit 113A has a certain rigidity in the axial direction, moves in the radial direction and has a spring property in the axial and radial directions. Also, since it is not necessary to form the spring parts 131, it is possible to easily manufacture the holder alignment unit 113A and to enhance the precision.

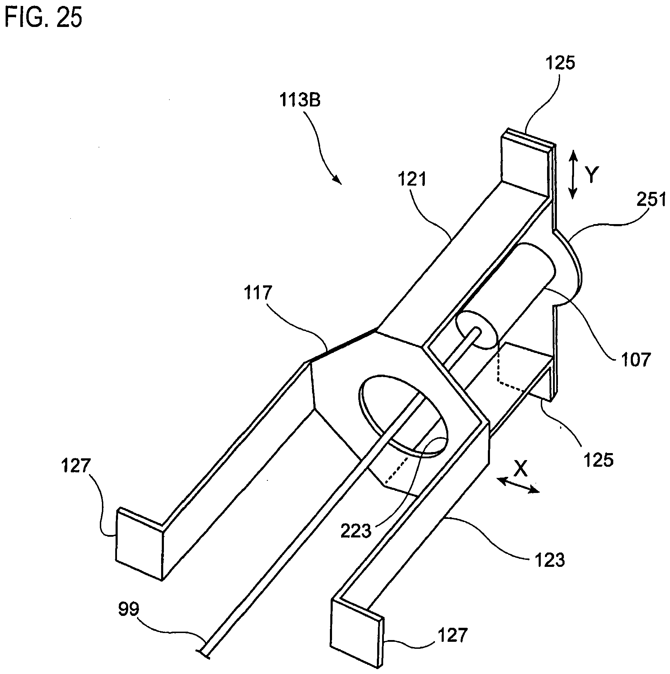

Fig. 25 is a perspective view of a holder alignment unit according to another modified embodiment in which the first linear support pieces 121 and the second linear support pieces 123 extend in opposite directions.

The holder alignment unit 113B according to this modified embodiment is obtained by further modifying the holder alignment unit 113A. The second linear support pieces 123, 123 protrude from the common plate part 117 in the direction opposite to the protrusion direction of the first linear support pieces 121, 121. With the holder alignment unit 113B according to this modified embodiment, a linear distance between fulcrum points (the second leading end portions 127) and points of action (the first leading end portions 125) can be ensured long, deformation can be caused more easily, and it becomes possible to perform the core alignment with a small reaction force.

According to the endoscope apparatus 100 having the above configuration, when the plug 95 begins to engage with the receptacle 93, positional deviations which may occur between the plug-side holders 105 and the receptacle-side holders 107 are removed by having the holder alignment units 113 move the receptacle-side holders 107 with respect to the floating base 109. In other words, the alignment is automatically performed in a direction in which the optical axes of the plug-side optical fiber 55 and the receptacle-side optical fiber 99 are matched. Thereby, the axes of the optical fibers 97, 99 are matched with high precision, so that it is possible to connect the optical fibers in low loss. As a result, it is possible to stably guide the laser lights.

In the endoscope apparatus 100, the plug-side optical fibers 55 and the receptacle-side optical fibers 99 are multi-mode fibers. However, since the holder alignment units 113 is provided, even for a connection structure using a single mode fiber, it is possible to perform a secure core alignment operation and to connect the cores with high positioning precision.

In the above illustrative embodiments, the connector has been described which connects the light source device 41 and the endoscope 11 of the endoscope apparatus 100. However, the invention is not limited to the illustrative embodiments. For example, the illustrative embodiments may be changed and/or modified by one skilled in the art based on the descriptions of the specification and well-known technologies, which are intended to be included in the scope of the invention to be protected. For example, the invention may be applied to various medical equipments such as rigid mirror, scope endoscope, a variety of surgery tools and the like.

As descried above, the specification discloses at least the followings:
(1) A medical equipment includes an optical connector that detachably connects a plug to a receptacle provided in an equipment main body to optically connect a plug-side optical fiber fixed to the plug and a receptacle-side optical fiber fixed to the receptacle. The plug includes a plug-side holder that maintains the first optical fiber. The receptacle includes a receptacle-side holder that holds the second optical fiber and a holder alignment unit that elastically supports the receptacle-side holder in an insertion direction of the plug and supports the receptacle-side holder so that the receptacle-side holder is movable in a direction perpendicular to the insertion direction. A plurality of engagement pairs of a protrusion and an engagement part are provided. The protrusion of each engagement pair is formed in one of the plug and the receptacle and protrudes in the insertion direction of the plug. The engagement part of each engagement pair is formed at in the other of the plug and the receptacle and is adapted to be engaged with the corresponding protrusion. Engagement beginning positions of the engagement pairs where the protrusions and the engagement parts begin to engage are different along the insertion direction of the plug. Each engagement pair has a narrower engagement gap between the protrusion and the engagement part than that between the protrusion and the engagement part of any pair which begin to engage earlier as the plug is inserted than the protrusion and the engagement part of each engagement pair begin to engage as the plug is inserted.
   With this medical equipment, when the optical connector is connected, the plurality of engagement pairs having the different engagement beginning positions are sequentially engaged. Therefore, it is possible to increase the positioning precision step by step. When the connection of the optical connector is completed, the optical fibers are automatically matched with high precision in terms of the optical axes thereof. Therefore, it is possible to simply connect the optical fibers in low loss.
(2) In the medical equipment of (1), the plug-side optical fiber and the receptacle-side optical fiber may be single mode fibers.
   With this medical equipment, even for a connection configuration using a single mode fiber in which the light is transmitted in a single mode, it is possible to perform a secure core alignment operation and to connect the cores with high positioning precision.
(3) In the medical equipment of any one of (1) to (2), plural sets of the plug-side holder and the receptacle-side holder may be provided. The optical connector may be adapted to detachably connect a plurality of plug-side optical fibers and a plurality of receptacle-side optical fibers.
   With this medical equipment, it is possible to detachably connect the plurality of optical fibers at a time.
(4) In the medical equipment of (1) to (3), the plug-side holder may include a plug-side ferrule and a plug-side inner sleeve. The plug-side ferrule fixes the plug-side optical fiber. The plug-side inner sleeve covers an outer circumference of the plug-side ferrule. The receptacle-side holder includes a receptacle-side ferrule and a receptacle-side inner sleeve. The receptacle-side ferrule fixes the receptacle-side optical fiber. The receptacle-side inner sleeve covers an outer circumference of the receptacle-side ferrule. An outer sleeve may be further provided which covers one of the plug-side inner sleeve and the receptacle-side inner sleeve and protrudes in the insertion direction of the plug. One of the plurality of engagement pairs may include the outer sleeve as the protrusion and includes the other of the plug-side inner sleeve, and the receptacle-side inner sleeve as the engagement part.
   With this medical equipment, it is possible to connect the inner sleeves with each other with high positioning precision by engaging the outer sleeves and the inner sleeves.
(5) In the medical equipment of (4), the plug-side holder may include a plug-side cylinder part that protrudes in the insertion direction of the plug further than the plug-side inner sleeve. The receptacle-side holder may include a receptacle-side cylinder part that protrudes in the insertion direction of the plug further than the receptacle-side inner sleeve and that has an inner circumference of a larger diameter than an outer diameter of the plug-side cylinder part. The plug-side cylinder part and the receptacle-side cylinder part may adapted to engage be with each other as one of the engagement pairs.
   With this medical equipment, it is possible to connect the inner sleeves with each other with high positioning precision by engaging the plug-side cylinder parts and the receptacle-side cylinder parts, after the holders are positioned. Also, since a rough positioning precision is required at an early engagement stage between the plug and the receptacle, it is possible to easily mount the plug to the receptacle.
(6) In the medical equipment of (5), the plug may include a plug-side outer cylinder that houses the plug-side holder therein and that protrudes in the insertion direction of the plug further than the plug-side holder. The receptacle may include a receptacle-side outer cylinder that houses the receptacle-side holder therein, that protrudes in the insertion direction of the plug further than the receptacle-side holder, and that has an inner circumference of a larger diameter than an outer diameter of the plug-side outer cylinder. The plug-side outer cylinder and the receptacle-side outer cylinder may be adapted to engage with each other as one of the engagement pairs.
   With this medical equipment, it is possible to connect the holders and the inner sleeves with each other with high positioning precision by engaging the plug-side outer cylinder and the receptacle-side outer cylinder, after the outer cylinders are positioned.
(7) In the medical equipment of any one of (1) to (6), a first graded index collimator may be incorporated into an optical connection end face part of the receptacle-side holder. The first graded index collimator may enlarge and collimate a beam diameter of light incident thereon from the receptacle-side optical fiber. The second graded index collimator may be incorporated into an optical connection end face part of the plug-side holder. The second graded index collimator has an approximately same core diameter as the first graded index collimator. The second graded index collimator may converge and introduce a beam diameter of light incident thereon from the first graded index collimator into the plug-side optical fiber.
   With the medical equipment, the beam diameter of the light emitted from the graded index collimator is enlarged, so that it is possible to improve a margin in the positioning precision between the plug and the receptacle.
(8) An endoscope apparatus is configured as the medical equipment of any one of (1) to (7). The endoscope apparatus includes an endoscope and a light source device. The endoscope irradiates light, which is introduced from the plug, from a leading end of an endoscope insertion portion that is adapted to be inserted into an object to be examined. The plug is connected to the light source device.
   With this endoscope apparatus, it is possible to simply generate the light for illumination of the endoscope by connecting the plug to the receptacle of the light source device.
(9) In the endoscope apparatus of (8), the light source device may supply a plurality of laser lights having different spectra to the endoscope.
   With the endoscope apparatus, by connecting the optical fibers in low loss, it is possible to surely supply the laser lights of different types.
(10) In the endoscope apparatus of (9), the light source device may supply the laser lights to the endoscope simultaneously or alternately.
   With this endoscope apparatus, by supplying the plurality of laser lights to the endoscope at any timing, it is possible to generate an optimized illumination pattern corresponding to an object to be observed by the endoscope and to thus improve the diagnosis precision of the endoscope.

## Claims

1. A medical equipment comprising:
an optical connector that detachably connects a plug (95) to a receptacle (93) provided in an equipment main body to optically connect a first, plug-side optical fiber (55) fixed to the plug (95) and a second, receptacle-side optical fiber (99) fixed to the receptacle (93), wherein the plug (95) includes a plug-side holder (105) that maintains the first optical fiber (55),
the receptacle (93) includes
a receptacle-side holder (107) that holds the second optical fiber (99), and
a holder alignment unit (113) that elastically supports the receptacle-side holder (107) in an insertion direction (a) of the plug (95) and supports the receptacle-side holder (107) so that the receptacle-side holder (107) is movable in a direction perpendicular to the insertion direction, a plurality of engagement pairs (145, 137; 149, 151), each consisting of a protrusion and an engagement part, wherein the protrusion (151, 145) of each engagement pair is formed in one of the plug (95) and the receptacle (93), and protrudes in the insertion direction (a) of the plug,
the engagement part (149, 137) of each engagement pair is formed in the other of the plug (95) and the receptacle (93), and is adapted to be engaged with the corresponding protrusion,
**characterized in that**
engagement beginning positions of the engagement pairs where the protrusions and the engagement parts begin to engage are different along the insertion direction of the plug (95), and
each engagement pair (145, 137; 149, 151) has a gap between its protrusion and its engagement part in the direction perpendicular to said insertion direction (a), wherein
(i) any engagement pair the protrusion and the engagement part of which begin to engage earlier has a wider gap, and
(ii) any engagement pair the protrusion and the engagement part of which begin to engage later has a narrower gap.

2. The medical equipment according to claim 1, wherein the plug-side optical fiber (55) and the receptacle-side optical fiber (99) are single mode fibers.

3. The medical equipment according to any one of claims 1 to 2, wherein plural sets of the plug-side holder (105) and the receptacle-side holder (107) are provided, and the optical connector is adapted to detachably connect a plurality of plug-side optical fibers (55) and a plurality of receptacle-side optical fibers (99).

4. The medical equipment according to any one of claims 1 to 3, wherein the plug-side holder (105) includes
a plug-side ferrule that fixes the plug-side optical fiber, and
a plug-side inner sleeve that covers an outer circumference of the plug-side ferrule, the receptacle-side holder includes
a receptacle-side ferrule that fixes the receptacle-side optical fiber, and
a receptacle-side inner sleeve that covers an outer circumference of the receptacle-side ferrule,
an outer sleeve is further provided which covers one of the plug-side inner sleeve and the receptacle-side inner sleeve and protrudes in the insertion direction of the plug, and
one of the plurality of engagement pairs includes
the outer sleeve as the protrusion and includes the other of the plug-side inner sleeve, and
the receptacle-side inner sleeve as the engagement part.

5. The medical equipment according to claim 4, wherein
the plug-side holder includes a plug-side cylinder part that protrudes in the insertion direction of the plug further than the plug-side inner sleeve,
the receptacle-side holder includes a receptacle-side cylinder part that protrudes in the insertion direction of the plug further than the receptacle-side inner sleeve and that has an inner circumference of a larger diameter than an outer diameter of the plug-side cylinder part, and
the plug-side cylinder part and the receptacle-side cylinder part are adapted to engage with each other as one of the engagement pairs.

6. The medical equipment according to claim 5, wherein
the plug includes a plug-side outer cylinder that houses the plug-side holder therein and that protrudes in the insertion direction of the plug further than the plug-side holder,
the receptacle includes a receptacle-side outer cylinder that houses the receptacle-side holder therein, that protrudes in the insertion direction of the plug further than the receptacle-side holder, and that has an inner circumference of a larger diameter than an outer diameter of the plug-side outer cylinder, and
the plug-side outer cylinder and the receptacle-side outer cylinder are adapted to engage with each other as one of the engagement pairs.

7. The medical equipment according to any one of claims 1 to 6, wherein
a first graded index collimator is incorporated into an optical connection end face part of the receptacle-side holder,
the first graded index collimator enlarges and collimates a beam diameter of light incident thereon from the receptacle-side optical fiber,
a second graded index collimator is incorporated into an optical connection end face part of the plug-side holder,
the second graded index collimator has an approximately same core diameter as the first graded index collimator, and
the second graded index collimator converges and introduces a beam diameter of light incident thereon from the first graded index collimator into the plug-side optical fiber.

8. An endoscope apparatus that is configured as the medical equipment according to any one of claims 1 to 7, the endoscope apparatus comprising:
an endoscope that irradiates light, which is introduced from the plug, from a leading end of an endoscope insertion portion that is adapted to be inserted into an object to be examined, and
a light source device to which the plug is connected.

9. The endoscope apparatus according to claim 8, wherein the light source device supplies a plurality of laser lights having different spectra to the endoscope.

10. The endoscope apparatus according to claim 9, wherein the light source device supplies the laser lights to the endoscope simultaneously or alternately.

## Patentansprüche

1. Medizinische Vorrichtung, umfassend:
einen optischen Verbinder, der lösbar einen Stecker (95) mit einer Buchse (93) an einem Vorrichtungshauptkörper verbindet, um eine erste, steckerseitige optische Faser (55), die an dem Stecker (95) fixiert ist, und eine zweite, buchsenseitige optische Faser (99), die an der Buchse (93) fixiert ist, optisch zu verbinden, wobei der Stecker (95) einen steckerseitigen Halter (105) enthält, der die erste optische Faser (55) hält, die Buchse (93) einen buchsenseitigen Halter (107) enthält, der die zweite optische Faser (99) hält, und
Halterausrichteinheit (113), welche den buchsenseitigen Halter (107) elastisch in einer Einführrichtung (a) des Steckers (95) abstützt und den buchsenseitigen Halter (107) derart abstützt, dass der buchsenseitige Halter (107) in einer Richtung rechtwinklig zur Einführrichtung bewegbar ist,
eine Mehrzahl von Eingriffspaaren (145, 137; 149, 151), jeweils bestehend aus einem Vorsprung und einem Eingriffsteil, wobei der Vorsprung (151, 145) jedes Eingriffspaars an dem einen Teil von dem Stecker (95) und der Buchse (93) ausgebildet ist und in die Einführrichtung (a) des Steckers vorsteht, wobei das Eingriffsteil (149, 137) jedes Eingriffspaars an dem anderen Teil von dem Stecker (95) und der Buchse (93) ausgebildet und dazu konfiguriert ist, um mit dem entsprechenden Vorsprung in Eingriff zu treten,
**dadurch gekennzeichnet, dass** die Eingriffs-Startpositionen der Eingriffspaare, bei denen die Vorsprünge und die Eingriffsteile miteinander in Eingriff zu treten beginnen, entlang der Einführrichtung des Steckers (95) unterschiedlich lang sind, und jedes Eingriffspaar (145, 137; 149, 151) zwischen seinem Vorsprung und seinem Eingriffsteil in der Richtung rechtwinklig zu der Einführrichtung (a) eine Lücke aufweist, wobei
(i) jedes Eingriffspaar, dessen Vorsprung und Eingriffsteil früher in Eingriff zu treten beginnen, eine größere Lücke besitzt, und
(ii) jedes Eingriffspaar, dessen Vorsprung und Eingriffsteil später in Eingriff zu treten beginnen, eine kleinere Lücke besitzt.

2. Vorrichtung nach Anspruch 1, bei der die steckerseitige optische Faser (55) und die buchsenseitige optische Faser (99) Einzelmodenfasem sind.

3. Vorrichtung nach einem der Ansprüche 1 bis 2, bei der mehrere Sätze aus dem steckerseitigen Halter (105) und dem buchsenseitigen Halter (107) vorgesehen sind, und der optische Verbinder dazu ausgebildet ist, mehrere steckerseitige optische Fasern (55) und mehrere buchsenseitige optische Fasern (99) lösbar zu verbinden.

4. Vorrichtung nach einem der Ansprüche 1 bis 3, bei der der steckerseitige Halter (105) enthält:
eine steckerseitige Zwinge, die die steckerseitige optische Faser fixiert, und
eine steckerseitige Innenhülse, die einen Außenumfang der steckerseitigen Zwinge bedeckt,
der buchsenseitige Halter enthält:
eine buchsenseitige Zwinge, welche die buchsenseitige optische Faser fixiert, und
eine buchsenseitige Innenhülse, die einen Außenumfang der buchsenseitigen Zwinge bedeckt,
wobei weiterhin eine Außenhülse vorgesehen ist, die eine von der steckerseitigen Innenhülse und der buchsenseitigen Innenhülse abdeckt und in Einführrichtung des Steckers vorsteht, und eines der mehreren Eingriffspaare enthält:
eine Außenhülse als den Vorsprung und die andere von der steckerseitigen Innenhülse, und
die buchsenseitige Innenhülse als das Eingriffsteil.

5. Vorrichtung nach Anspruch 4, bei der
der steckerseitige Halter einen steckerseitigen Zylinderteil enthält, der in Einführrichtung des Steckers weiter vorsteht als die steckerseitige Innenhülse,
der buchsenseitige Halter einen buchsenseitigen Zylinderteil enthält, der in Einführrichtung des Steckers weiter vorsteht als die buchsenseitige Innenhülse, und die einen Innenumfang mit einem größeren Durchmesser besitzt, als es dem Außendurchmesser des steckerseitigen Zylinderteils entspricht, und
der steckerseitige Zylinderteil und der buchsenseitige Zylinderteil dazu ausgebildet sind, miteinander als eins der Eingriffspaare ineinander zu greifen.

6. Vorrichtung nach Anspruch 5, bei der
der Stecker einen steckerseitigen Außenzylinder enthält, der den steckerseitigen Halter in sich aufnimmt, und der in Einführrichtung des Steckers weiter vorsteht als der steckerseitige Halter,
die Buchse einen buchsenseitigen Außenzylinder enthält, der in sich den buchsenseitigen Halter aufnimmt und in Einführrichtung des Steckers weiter vorsteht als der buchsenseitige Halter, und der einen Innenumfang mit einem größeren Durchmesser als der Außendurchmesser des steckerseitigen Außenzylinders aufweist, und
der steckerseitige Außenzylinder und der buchsenseitige Außenzylinder dazu ausgebildet sind, miteinander als eins der Eingriffspaare zusammenzuwirken.

7. Vorrichtung nach einem der Ansprüche 1 bis 6, bei der
in eine optische Verbindungsstimfläche des buchsenseitigen Halters ein erster Gradientenindex-Kollimator eingebaut ist,
der erste Gradientenindex-Kollimator einen Strahldurchmesser des auf ihn von der buchsenseitigen optischen Faser auftreffenden Lichts vergrößert und kollimiert,
ein zweiter Gradientenindex-Kollimator in eine optische Verbindungsstimfläche des steckerseitigen Halters eingebaut ist,
der zweite Gradientenindex-Kollimator einen näherungsweise gleich großen Kerndurchmesser wie der erste Gradientenindex-Kollimator besitzt, und
der zweite Gradientenindex-Kollimator einen Strahldurchmesser des auf ihn von dem ersten Gradientenindex-Kollimator auftreffenden Lichts in die steckerseitige optische Faser konvergiert und einführt.

8. Endoskopvorrichtung, konfiguriert als medizinische Vorrichtung nach einem der Ansprüche 1 bis 7, umfassend:
ein Endoskop, welches Licht abstrahlt, welches aus dem Stecker von einem Vorderende des Endoskopfeinführabschnitts eingeleitet wurde, der ausgebildet ist zum Einführen in ein Untersuchungsobjekt, und
eine Lichtquelleneinrichtung, an die der Stecker angeschlossen ist.

9. Endoskopvorrichtung nach Anspruch 8, bei der die Lichtquelleneinrichtung mehrere Arten von Laserlicht mit unterschiedlichen Spektren dem Endoskop zuführt.

10. Endoskopvorrichtung nach Anspruch 9, bei der die Lichtquelleneinrichtung die Laserstrahlen simultan oder abwechselnd in das Endoskop einbringt.

## Revendications

1. Équipement médical, comprenant :
un connecteur optique, lequel connecte de manière amovible une fiche (95) à un réceptacle (93) prévu dans un corps principal d'équipement, afin de connecter par voie optique une première fibre optique côté fiche (55) fixée sur la fiche (95) et une seconde fibre optique côté réceptacle (99) fixée sur le réceptacle (93), dans lequel la fiche (95) inclut un support côté fiche (105), lequel maintient la première fibre optique (55) ; le réceptacle (93) inclut un support côté réceptacle (107), lequel tient la seconde fibre optique (99), et une unité d'alignement de support (113), laquelle supporte de manière élastique le support côté réceptacle (107) dans une direction d'introduction (a) de la fiche (95) et supporte le support côté réceptacle (107), de sorte que le support côté réceptacle (107) est mobile dans une direction perpendiculaire à la direction d'introduction ; une pluralité de paires de prise (145, 137; 149, 151), chacune consistant en une protubérance et une partie de prise, dans lequel la protubérance (151, 145) de chaque paire de prise est formée dans un élément parmi la fiche (95) et le réceptacle (93), et fait saillie dans la direction d'introduction (a) de la fiche ; la partie de prise (149, 137) de chaque paire de prise est formée dans l'autre élément parmi la fiche (95) et le réceptacle (93), et est apte à venir en prise avec la protubérance correspondante,
**caractérisé en ce que**
des positions de début de prise des paires de prise, où les protubérances et les parties de prise commencent à venir en prise, sont différentes le long de la direction d'introduction de la fiche (95), et
chaque paire de prise (145, 137; 149, 151) présente un espace entre sa protubérance et sa partie de prise dans la direction perpendiculaire à ladite direction d'introduction (a), dans lequel
(i) l'une quelconque des paires de prise dont la protubérance et la partie de prise commence à venir en prise plus tôt présente un espace plus large, et
(ii) l'une quelconque des paires de prise dont la protubérance et la partie de prise commence à venir en prise plus tard présente un espace plus étroit.

2. Équipement médical selon la revendication 1, dans lequel la fibre optique côté fiche (55) et la fibre optique côté réceptacle (99) sont des fibres monomodales.

3. Équipement médical selon l'une quelconque des revendications 1 à 2, dans lequel plusieurs jeux du support côté fiche (105) et du support côté réceptacle (107) sont prévus, et le connecteur optique est apte à connecter de manière amovible une pluralité de fibres optiques côté fiche (55) et une pluralité de fibres optiques côté réceptacle (99).

4. Équipement médical selon l'une quelconque des revendications 1 à 3, dans lequel
le du support côté fiche (105) inclut ;
une ferrule côté fiche, laquelle fixe la fibre optique côté fiche,
un manchon intérieur côté fiche, lequel recouvre une circonférence extérieure de la ferrule côté fiche ;
le support côté réceptacle inclut :
une ferrule côté réceptacle, laquelle fixe la fibre optique côté réceptacle, et
une manchon intérieur côté réceptacle, lequel recouvre une circonférence extérieure de la ferrule côté réceptacle ;
un manchon extérieur est en outre prévu, lequel recouvre un élément parmi le manchon intérieur côté fiche et le manchon intérieur côté réceptacle et fait saillie dans la direction d'introduction de la fiche, et
une paire parmi la pluralité de paires de prise inclut :
le manchon extérieur comme protubérance, et inclut l'autre élément parmi le manchon intérieur côté fiche, et le manchon intérieur côté réceptacle comme partie de prise.

5. Équipement médical selon la revendication 4, dans lequel
le support côté fiche inclut une partie de cylindre côté fiche, laquelle fait saillie dans la direction d'introduction de la fiche plus loin que le manchon intérieur côté fiche ;
le support côté réceptacle inclut une partie de cylindre côté réceptacle, laquelle fait saillie dans la direction d'introduction de la fiche plus loin que le manchon intérieur côté réceptacle, et laquelle présente une circonférence intérieure d'un diamètre plus grand qu'un diamètre extérieur de la partie de cylindre côté fiche, et
la partie de cylindre côté fiche et la partie de cylindre côté réceptacle sont aptes à venir en prise l'une avec l'autre sous la forme d'une des paires de prise.

6. Équipement médical selon la revendication 5, dans lequel
la fiche inclut un cylindre extérieur côté fiche, lequel loge le support côté fiche à l'intérieur, et lequel fait saillie dans la direction d'introduction de la fiche plus loin que le support côté fiche,
le réceptacle inclut un cylindre extérieur côté réceptacle, lequel loge le support côté réceptacle à l'intérieur, lequel fait saillie dans la direction d'introduction de la fiche plus loin que le support côté réceptacle, et lequel présente une circonférence intérieure d'un diamètre plus grand qu'un diamètre extérieur du cylindre extérieur côté fiche, et
le cylindre extérieur côté fiche et le cylindre extérieur côté réceptacle sont aptes à venir en prise l'un avec l'autre sous la forme de l'une des paires de prise.

7. Équipement médical selon l'une quelconque des revendications 1 à 6, dans lequel
un premier collimateur à gradient d'indice est intégré dans une partie de face d'extrémité de connexion optique du support côté réceptacle ;
le premier collimateur à gradient d'indice agrandit et collimate un diamètre de faisceau de la lumière incidente sur lui à partir de la fibre optique côté réceptacle ;
un second collimateur à gradient d'indice est intégré dans une partie de face d'extrémité de connexion optique du support côté fiche ;
le second collimateur à gradient d'indice présente un diamètre de coeur approximativement identique à celui du premier collimateur à gradient d'indice, et
le second collimateur à gradient d'indice converge et introduit un diamètre de faisceau de la lumière incidente sur lui à partir du premier collimateur à gradient d'indice dans la fibre optique côté fiche.

8. Appareil d'endoscope configuré sous la forme de l'équipement médical selon l'une quelconque des revendications 1 à 7, l'appareil d'endoscope comprenant :
un endoscope, lequel irradie avec une lumière, introduit à partir de la fiche, d'une extrémité avant d'une portion d'introduction d'endoscope, laquelle est apte à être introduite dans un objet à examiner, et
un dispositif de source lumineuse auquel la fiche est connectée.

9. Appareil d'endoscope selon la revendication 8, dans lequel le dispositif de source lumineuse fournit une pluralité de lumières laser présentant des spectres différents à l'endoscope.

10. Appareil d'endoscope selon la revendication 9, dans lequel le dispositif de source lumineuse fournit les lumières laser à l'endoscope simultanément ou par alternance.
